# EUROPÄISCHE PATENTANMELDUNG

(11) **EP 0 805 209 A2**
(43) Veröffentlichungstag der Anmeldung: **05.11.1997**
(21) Anmeldenummer: 97106531.3
(22) Anmeldetag: 21.04.1997
(51) Int. Cl.: C12N 15/86, C12N 15/62

(54) **Nukleinsäurekonstrukte mit Genen kodierend für Transportsignale**

(30) Priorität: 03.05.1996 DE 19617851
(71) Anmelder: HOECHST AKTIENGESELLSCHAFT, 65929 Frankfurt am Main (DE)
(72) Erfinder: Sedlacek, Hans-Harald, Prof. Dr., 35041 Marburg (DE); Müller, Rolf, Prof. Dr., 35037 Marburg (DE); Lührmann, Reinhard, Prof. Dr., 35041 Marburg (DE)

(57) **Zusammenfassung**

Offenbart werden Nukleinsäurekonstrukte, die ein nukleäres Retentions-Signal aufweisen, das in Leserichtung stromabwärts mit einem Transgen verknüpft ist. Durch das nukleäre Retentions-Signal kann die Anwesenheit des Transkriptionsproduktes im Zellkern oder auch der intrazelluläre Transport des Transkriptionsproduktes gesteuert werden.

SEQUENZPROTOKOLL

(1) ALLGEMEINE INFORMATION:

(i) ANMELDER:
(A) NAME: Hoechst Aktiengesellschaft
(B) STRASSE: -
(C) ORT: Frankfurt
(D) BUNDESLAND: -
(E) LAND: Deutschland
(F) POSTLEITZAHL: 65926
(G) TELEPHON: 069-305-3005
(H) TELEFAX: 069-35-7175
(I) TELEX: 412 34 700 ho d

(ii) ANMELDETITEL: Nukleinsaeurekonstrukte mit Genen kodierend
fuer Transportsignale

(iii) ANZAHL DER SEQUENZEN: 9

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 41 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..41

(xi) SEQUENZBESCHREIBUNG: SEQ ID NO: 1:

AGCTTCCGCA TGCTGATGAG GCCGCAAGGC CGAAACGGCA G 41

(2) INFORMATION ZU SEQ ID NO: 2:

(i) SEQUENZ CHARAKTERISTIKA:
(A) LÄNGE: 41 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..41

(xi) SEQUENZBESCHREIBUNG: SEQ ID NO: 2:

AATTCTGCCG TTTCGGCCTT GCGGCCTCAT CAGCATGCGG A 41

(2) INFORMATION ZU SEQ ID NO: 3:

(i) SEQUENZ CHARAKTERISTIKA:
(A) LÄNGE: 40 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..40

(xi) SEQUENZBESCHREIBUNG: SEQ ID NO: 3:

AGCTTCCAGC CTGATGAGGC CGCAAGGCCG AAACAGGAAG 40

(2) INFORMATION ZU SEQ ID NO: 4:

(i) SEQUENZ CHARAKTERISTIKA:
(A) LÄNGE: 40 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..40

(xi) SEQUENZBESCHREIBUNG: SEQ ID NO: 4:

AATTCTTCCT GTTTCGGCCT TGCGGCCTCA TCAGGCTGGA 40

(2) INFORMATION ZU SEQ ID NO: 5:

(i) SEQUENZ CHARAKTERISTIKA:
(A) LÄNGE: 38 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..38

(xi) SEQUENZBESCHREIBUNG: SEQ ID NO: 5:

AGCTTGCACA CTGATGAGGC CGCAAGGCCG AAACTGCG 38

(2) INFORMATION ZU SEQ ID NO: 6:

(i) SEQUENZ CHARAKTERISTIKA:
(A) LÄNGE: 38 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..38

(xi) SEQUENZBESCHREIBUNG: SEQ ID NO: 6:

AATTCGCAGT TTCGGCCTTG CGGCCTCATC AGTGTGCA 38

(2) INFORMATION ZU SEQ ID NO: 7:

(i) SEQUENZ CHARAKTERISTIKA:
(A) LÄNGE: 44 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..44

(xi) SEQUENZBESCHREIBUNG: SEQ ID NO: 7:

AGCTTGAAGT TTATACTGAT GAGGCCGCAA GGCCGAAACT TCAG 44

(2) INFORMATION ZU SEQ ID NO: 8:

(i) SEQUENZ CHARAKTERISTIKA:
(A) LÄNGE: 44 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..44

(xi) SEQUENZBESCHREIBUNG: SEQ ID NO: 8:

AATTCTGAAG TTTCGGCCTT GCGGCCTCAT CAGTATAAAC TTCA 44

(2) INFORMATION ZU SEQ ID NO: 9:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 9:

TACTGTATGT ACATACAGTA 20

## Beschreibung

Gegenstand der vorliegenden Anmeldung sind Nukleinsäurekonstrukte, die in der Gentechnologie und insbesondere in der Prophylaxe oder Therapie von Erkrankungen (im nachfolgenden Gentherapie genannt) verwendet werden können.

Bei der Gentherapie werden Gene in den Organismus eingeführt, die im Organismus exprimiert werden sollen. Die Regulation der Expression dieser Gene ist bedeutsam für den prophylaktischen oder therapeutischen Effekt der Gentherapie.

In den Patentanmeldungen PCT/GB95/02000, PCT/EP 95/03370, PCT/EP 95/03371, PCT/EP 95/03368, PCT/EP 95/03339 werden Regulatoren für die Expression eines Genes beschrieben. Diese Regulatoren bestehen aus einer Aktivatorsequenz, deren Funktion beispielsweise die zellspezifische oder virusspezifische Aktivierung der basalen Transkription ist. Die DNA-Sequenz dieser Aktivatorsequenz ist mit ihrem 3'-Ende an das 5'-Ende eines Promotormoduls verknüpft. An das 3'-Ende des Promotormoduls ist wiederum mit seinem 5'-Ende das Strukturgen geknüpft.

Das Promotormodul besteht aus Nukleinsäuresequenzen zur Bindung der Transkriptionsfaktoren der Familien CDF und CHF oder E2F und CHF. Diese Bindung führt in der G0- und G1-Phase des Zellzyklus zu einer Hemmung der stromaufwärts gelegenen Aktivatorsequenz und damit zu einer Inhibition der Transkription des stromabwärts (d.h. in Richtung der Transkription) gelegenen Strukturgenes.

In der G0- und G1-Phase der Zellteilung liegt der DNA-Gehalt der Zelle im diploiden Zustand vor. In der G0-Phase ist die Zelle in Ruhe, in der G1-Phase ist sie in ihrer Zellzyklusprogression inhibiert. An die G1-Phase schließt sich die S-Phase an, in der die DNA-Synthese stattfindet und in der das Genom repliziert wird. Nachfolgend schließt sich die G2-Phase an, in der die Zelle im tetraploiden Zustand ist. Auf die G2-Phase folgt die Zellteilung (Mitose = M-Phase). Die Tochterzellen kommen in den G0- oder G1-Zustand.

Die Kombination einer zellspezifischen oder virusspezifischen Aktivatorsequenz mit einem diese Aktivatorsequenz in der G0- und G1-Phase hemmenden Promotormodul ermöglicht somit die zellspezifische oder virusspezifische als auch zellzyklusspezifische (d.h. auf die S- und G2-Phase beschränkte) Regulation der Expression eines Strukturgenes.

Die Kombination einer Aktivatorsequenz mit einem Promotormodul wird chimärer Promotor genannt. Es gibt zahlreiche Anwendungsmöglichkeiten für chimäre Promotoren in der Gentherapie, jedoch auch eine Reihe von durch Unzulänglichkeiten bedingte Einschränkungen.

Beispiele für diese Einschränkungen sind
- eine schwache Aktivatorsequenz, die eine zu geringe Transkription des Strukturgenes bewirkt,
- die Verwendung einer Aktivatorsequenz, die durch das gewählte Promotormodul nicht ausreichend zellzyklusabhängig gehemmt werden kann,
- die Beschränkung auf zwei (zum Beispiel zell- oder virusspezifische und zellzyklusspezifische) Regulatoren der Transkription des Strukturgenes und
- ein mangelhafter Verbleib des Transkriptionsproduktes im Zellkern
- oder der mangelhafte intrazelluläre Transport des Transkriptionsproduktes des in die Zelle eingeführten Strukturgenes.

Es ist eine Aufgabe der vorliegenden Erfindung, Nukleinsäurekonstrukte zur Verfügung zu stellen, die eine präzise Regulierung der Expression von fremden Genen (Transgene) in den Wirtszellen ermöglichen. Gegenstand der vorliegenden Erfindung sind daher Nukleinsäurekonstrukte, die ein nukleäres Retentions-Signal aufweisen, daß in Leserichtung stromabwärts mit einem Transgen verknüpft ist.

Bevorzugt umfassen die Nukleinsäurekonstrukte wenigstens folgende Komponenten, und zwar in Leserichtung vom 5'-Ende zum 3'-Ende:
a) eine erste unspezifische, zellspezifische, virusspezifische, metabolisch und/oder zellzyklusspezifisch aktivierbare Promotor- oder Enhancersequenz (I), die die basale Transkription eines Transgenes aktiviert,
b) ein Transgen, das als Strukturgen für einem Wirkstoff kodiert,
c) ein nukleäres Retentions-Signal, dessen cDNA am 5'-Ende mit dem 3'-Ende des Strukturgenes (b) mittelbar oder unmittelbar verknüpft ist.

Bevorzugterweise hat das Transkriptionsprodukt des nukleären Retentions-Signals eine Bindestruktur für einen nukleären Export-Faktor.

Die erfindungsgemäßen Nukleinsäurekonstrukte können zusätzlich zu den Komponenten a) bis c) folgende Komponenten aufweisen:
d) eine weitere Promotor- oder Enhancersequenz (II), welche die basale Transkription eines nukleären Export-Faktores aktiviert und
e) eine Nukleinsäure kodierend für einen nukleären Export-Faktor, der an das Transkriptionsprodukt des nukleären Retentions-Signals (c) bindet und hierdurch den Transport des Transkriptionsproduktes des Transgens aus dem Zellkern vermittelt.

Die erste (I) Promotor- oder Enhancersequenz (a) und die zweite (II) Promotor- oder Enhancersequenz (d) können gleich oder unterschiedlich sein und, mindestens eine der Komponenten a) und d) kann unspezifisch, zellspezifisch, virusspezifisch, metabolisch, insbesondere durch Hypoxie oder zellzyklusspezifisch aktivierbar sein.

Im Rahmen der vorliegenden Erfindung kann wenigstens eine der Promotor- oder Enhancersequenzen (a) und (d) ein chimärer Promotor sein, bei welchem das Promotormodul CDE-CHR oder E2FBS-CHR mit einer stromaufwärts benachbarten zellspezifisch, virusspezifisch oder metabolisch aktivierbaren Aktivatorsequenz interagieren und dadurch die Expression eines stomabwärts gelegenen Gens beeinflussen, insbesondere inhibieren kann.

Bei den Komponenten a) und d) kann es sich um eine Aktivator-responsive Promotoreinheit handeln. Derartige Konstrukte weisen weiterhin folgende Komponenten auf:
f) wenigstens eine Promotor- oder Enhancersequenz, die unspezifisch, virusspezifisch, metabolisch oder zellspezifisch und/oder zellzyklusspezifisch aktivierbar ist
g) wenigstens eine Aktivatorsubeinheit, die stromabwärts von der Promotor- oder Enhancersequenz (f) gelegen ist und durch die Promotor- oder Enhancersequenz (f) in ihrer basalen Transkription aktiviert wird, und
h) einen Aktivator-responsiven Promotor, welcher durch die Expressionsprodukte einer oder mehrerer Aktivatorsubeinheit(en) (g) aktiviert wird.

In einer Ausführungsform der Erfindung handelt es sich um Nukleinsäurekonstrukte, bei welchen die Promotor- oder Enhancersequenz (a) und/oder (d) und/oder der Aktivator-responsive Promotor ein chimärer Promotor ist und die Aktivatorsubeinheit (g) ein Gen für wenigstens einen Transkriptionsfaktor darstellt, der den chimären Promotor des Aktivator-responsiven Promotors (h) aktiviert.

Ein Beispiel für einen Aktivator-responsiven Promotor aktiviert durch zwei Aktivatorsubeinheiten (g, g') (h) stellt der LexA-Operator in Verbindung mit dem SV40-Promotor dar. Die Aktivatorsubeinheit (g) umfaßt die cDNA für das LexA-DNA-Bindeprotein kodierend für die Aminosäuren 1-81 oder 1-202, deren 3'-Ende verknüpft ist mit dem 5'-Ende der cDNA für das Gal80-Protein (Aminosäuren 1-435). Die zweite Aktivatorsubeinheit (g') umfaßt die cDNA der Gal80-Bindungsdomäne des Gal4-Proteins kodierend für die Aminosäuren 851-881, deren 3'-Ende verknüpft ist mit dem 5'-Ende der cDNA des SV40 large T-Antigens kodierend für die Aminosäuren 126-132, deren 3'-Ende verknüpft ist mit dem 5'-Ende der cDNA für die Transaktivierungsdomäne des VP16 von HSV-1 kodierend für die Aminosäuren 406-488.

Ein weiteres Beispiel für einen Aktivator-responsiven Promotor aktiviert durch zwei Aktivatorsubeinheiten (g, g') stellt die Bindesequenz für das Gal4-Protein in Verbindung mit dem SV40-Promotor dar.

Die Aktivierungseinheit (g) umfaßt die cDNA für die DNA-Bindedomäne des Gal4-Proteins (Aminosäuren 1-147), deren 3' Ende verknüpft ist mit dem 5' Ende der cDNA für das Gal80-Protein (Aminosäuren 1-435).

Die zweite Aktivierungssubeinheit (g') umfaßt die cDNA für die Gal80-Bindungsdomäne von Gal4 (Aminosäuren 851 bis 881), deren 3' Ende verknüpft ist mit dem 5' Ende der cDNA des nuklearen Lokalisationssignals von SV40 (SV40 Large T, Aminosäuren 126 bis 132), deren 3' Ende verknüpft ist mit dem 5' Ende der cDNA für die Transaktivierungsdomäne des VP16 von HSV-1 kodierend für die Aminosäuren 406-488.

Ein weiteres Beispiel für zwei Aktivatorsubeinheiten (g, g'), die den Aktivatorresponsiven Promotor, bestehen aus der Bindesequenz für das Gal4-Protein und dem SV40-Promotor aktivieren, stellt dar
- die Aktivierungseinheit (g) umfassend die cDNA für die zytoplasmische Domäne des CD4-T-Zellantigens (Aminosäuren 397-435), deren 5' Ende verknüpft ist mit dem 3' Ende der cDNA für die Transaktivierungsdomäne des VP16 von HSV-1 (Aminosäuren 406-488), deren 5' Ende wiederum verknüpft ist mit dem 3' Ende der cDNA des nuklearen Lokalisationssignals von SV40 (SV40 Large T, Aminosäuren 16 bis 132) und
- die Aktivierungseinheit (g 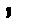 ) umfassend die cDNA des nuklearen Lokalisationssignals von SV40 (SV40 Large T, Aminosäuren 126 bis 132); cDNA für die DNA Bindedomäne des Gal4-Proteins (Aminosäuren 1-147), deren
   3' Ende verknüpft ist mit dem 5' Ende der cDNA für die CD4 Bindesequenz des p56 lck Proteins (Aminosäuren 1-71).

Bevorzugt wird das Gen kodierend für das nukleäre Retentions-Signal ausgewählt aus der Gruppe umfassend das Rev-responsive Element (RRE) von HIV-1 oder HIV-2, das RRE-äquivalente Retentions-Signal von Retroviren oder das RRE-äquivalente Retentions-Signal des HBV.

Der nukleäre Export-Faktor (e) ist bevorzugterweise ein Gen ausgewählt aus der Gruppe umfassend das Rev-Gen der Viren HIV-1, HIV-2, Visna-Maedi Virus, Caprine arthritis encephalitis Virus, das Virus der infektiösen Anämie des Pferdes, das Immundefizienzvirus der Katze, von Retroviren, von HTLV oder das Gen des hnRNP-A1-Proteins oder das Gen des Transkriptionsfaktors TFIII-A. In der Regel handelt es sich bei der Nukleinsäure um DNS. Die erfindungsgemäßen Nukleinsäurekonstrukte werden üblicherweise als Vektoren, insbesondere Plasmidvektoren oder virale Vektoren eingesetzt.

Bei dem Transgen handelt es sich in der Regel um Strukturgene, die für einen pharmakologisch aktiven Wirkstoff kodieren, der ausgewählt ist aus der Gruppe umfassend Cytokine, Wachstumsfaktoren, Antikörper oder Antikörperfragmente, Fusionsproteine zwischen Liganden wie z.B. Antikörper oder Antikörperfragmente und Cytokinen oder Wachstumsfaktoren, Rezeptoren für Cytokine oder Wachstumsfaktoren, antiproliferativ oder zytostatisch wirkende Proteine, Angiogeneseinhibitoren, Gerinnungsfaktoren, Thrombose-induzierende Substanzen und Gerinnungshemmer, fibrinolytisch wirkende Substanzen, Komplement aktivierende Proteine, Virushüllproteine, bakterielle Antigene und parasitäre Antigene, auf den Blutkreislauf wirkende Proteine und Ribozyme.

Bevorzugterweise handelt es sich bei dem Transgen um ein Strukturgen, das für ein Ribozym kodiert, welches die mRNA inaktiviert, welche kodiert für ein Protein ausgewählt aus der Gruppe umfassend Zellzykluskontrollproteine, insbesondere Cyclin A, Cyclin B, Cyclin D1, Cyclin E, E2F1-5, cdc2, cdc25C oder DP1, oder Virusproteine oder Cytokine oder Wachstumsfaktoren oder deren Rezeptoren.

In einer besonderen Ausführungsform kann es sich bei dem Transgen um ein Stukturgen handeln, welches für ein Protein kodiert, das einen kontrollierten Zelltod (Apoptose) auslöst, insbesondere Sphingomyelinase.

In einer anderen Ausführungsform kann es sich bei dem Transgen (b) um ein Strukturgen handeln, daß für ein Enzym kodiert, welches eine Vorstufe eines Pharmakons in ein Pharmakon spaltet. In einer weiteren Ausführungsform kann das Strukturgen für ein Ligand-Enzymfusionsprotein kodieren, wobei das Enzym eine Vorstufe eines Pharmakons in ein Pharmakon spaltet und der Ligand an eine Zelloberfläche bindet, bevorzugt an Endothelzellen oder Tumorzellen.

Die Promotor-, Enhancer- oder Aktivatorsequenz kann ausgewählt sein aus der Gruppe von genregulatorischen Nukleotidsequenzen aktiviert in Endothelzellen, glatten Muskelzellen, quergestreiften Muskelzellen, Makrophagen, Lymphozyten, Tumorzellen, Leberzellen, Leukämiezellen und Gliazellen oder von Promotorsequenzen der Viren HBV, HCV, HSV, HPV, EBV, HTLV oder HIV.

Die Erfindung betrifft auch isolierte Zellen oder Zellinien, die ein erfindungsgemäßes Nukleinsäurekonstrukt enthalten.

Derartige Zellen können zur Bereitstellung eines Heilmittels zur Behandlung einer Erkrankung verwendet werden, wobei die Bereitstellung des Heilmittels die Einführung des Nukleinsäurekonstrukts in eine Zielzelle umfaßt. Derartige Erkrankungen gehen häufig mit übermäßiger Zellvermehrung einher.

Zur Bereitstellung des Heilmittels muß zunächst ein Nukleinsäurekonstrukt in die Zielzelle eingeführt werden.

Die erfindungsgemäßen Nukleinsäurekonstrukte erlauben es, beliebige Promotoren, Enhancer oder Aktivatorsequenzen zu verwenden und insbesondere den Verbleib des Transkriptionsproduktes des in die Zelle eingeführten Strukturgenes im Zellkern zu verstärken oder den intrazellulären Transport des Transkriptionsproduktes des in die Zelle eingeführten Strukturgenes zu erhöhen.

Das erfindungsgemäße Nukleinsäurekonstrukt für einen verstärkten Verbleib des Transkriptionsproduktes im Zellkern umfaßt folgende Komponenten:
a) eine Promotor- oder Enhancersequenz I, welche die basale Transkription des Strukturgenes aktiviert,
b) ein Transgen (Strukturgen), das bevorzugt in Form einer cDNA vorliegt, welches für den gewünschten Wirkstoff kodiert, und
c) ein nukleäres Retentions-Signal (NRS), dessen cDNA am 5'-Ende bevorzugt unmittelbar mit dem 3'-Ende des Strukturgenes verknüpft ist.

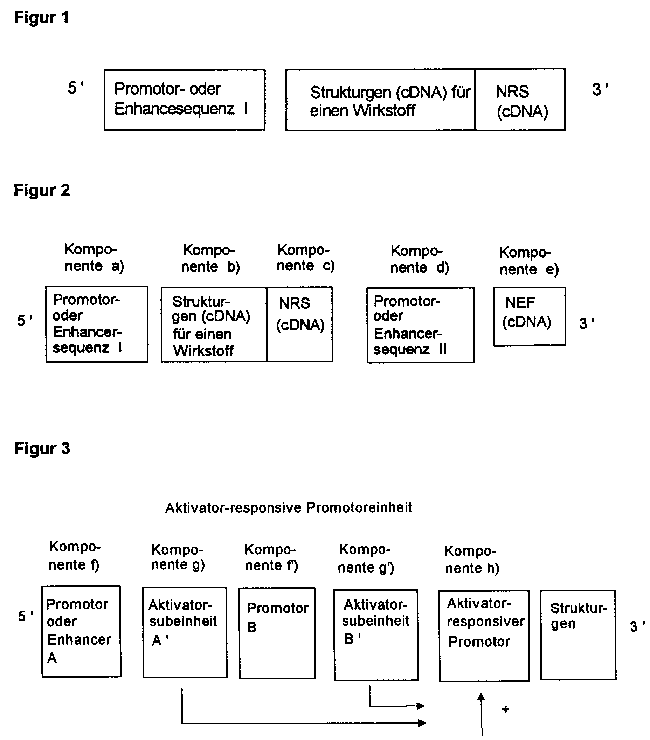

Die Anordnung der einzelnen Komponenten ist beispielsweise in Figur 1 wiedergegeben.

Das erfindungsgemäße Nukleinsäurekonstrukt für den verstärkten intrazellulären Transport des Transkriptionsproduktes weist neben einer Promotor- oder Enhancersequenz I, welche die basale Transkription des Strukturgenes aktiviert, und einem Transgen, welches für den gewünschten Wirkstoff kodiert, ein nukleäres Retentions-Signal (NRS) auf, dessen cDNA am 5'-Ende mit dem 3'-Ende des Strukturgenes verknüpft ist und dessen messenger RNA Bindestruktur für einen nukleären Export-Faktor (NEF) ist.

Darüber hinaus besitzen derartige Nukleinsäurekonstrukte eine weitere Promotor- oder Enhancersequenz (II), welche die basale Transkription der cDNA des nukleären Export-Faktors (NEF) aktiviert und dessen Expressionsprodukt an das nukleäre Retentions-Signal (NRS) bindet und hierdurch den Transport des Transkriptionsproduktes des Strukturgenes aus dem Zellkern vermittelt.

Die Anordnung der einzelnen Komponenten ist beispielsweise in Figur 2 wiedergegeben.

Bei den erfindungsgemäßen Nukleinsäurekonstrukten können die Promotor- oder Enhancersequenzen der Komponenten a) oder d) gleich oder unterschiedlich sein, des weiteren können die Komponenten d) und e) stromaufwärts oder stromabwärts der Komponenten a), b) und c) gelegen sein.

Als Promotor- oder Enhancersequenz wird bevorzugt wenigstens eine starke Promotor- oder Enhancersequenz, wie beispielsweise vom CMV (EP-A-0173177) oder SV40 oder jede andere, dem Fachmann bekannte Promotor- oder Enhancersequenz, verwendet.

In einer bevorzugten Ausführungsform ist bei den erfindungsgemäßen Nukleinsäurekonstrukten mindestens eine Promotor- oder Enhancersequenz zellspezifisch, metabolisch (z.B. durch Hypoxie), virusspezifisch oder zellzyklusspezifisch aktivierbar.

Besonders bevorzugt werden
- diejenigen Promotor- oder Enhancersequenzen, die die Transkription zellspezifisch in Endothelzellen, glatte Muskelzellen, quergestreifte Muskelzellen, blutbildende Zellen, Lymphozyten, Makrophagen, Gliazellen oder Tumorzellen aktivieren und/oder
- Promotorsequenzen bzw. Enhancersequenzen der Viren HBV, HCV, HSV, HPV, CMV, EBV, HTLV oder HIV und/oder
- Promotor- oder Enhancersequenzen, welche metabolisch aktivierbar sind wie beispielsweise der durch Hypoxie induzierbare Enhancer (Semenza et al., PNAS 88, 5680 (1991)) oder Promotor (Mc Burney et al., Nucleic Acids Res. 19, 5755 (1991); WO 95/21927) und/oder
- Promotoren, welche zellzyklusspezifisch aktivierbar sind wie beispielsweise der Promotor des cdc25C Genes, des Cyclin A Genes, des cdc2 Genes (Lucibello et al., EMBO J. 14, 132 (1995), Zwicker et al., EMBO J. 14, 4514 (1995), Zwicker et al., Nucl. Acids Res. 23, 2833 (1995)), des B-myb Genes (Lam et al., EMBO J. 12, 2705 (1993)), des DHFR-Genes (Means et al., Mol. Cell Biol. 12, 1054 (1992) und des E2F-1 Genes (Johnson et al., Genes Dev. 8, 1514 (1994), Hsiao et al., Genes Dev. 8, 15256 (1994)).

In einer weiteren bevorzugten Ausführungsform ist bei den erfindungsgemäßen Nukleinsäurekonstrukten mindestens eine Promotor- oder Enhancersequenz ein chimärer Promotor. Im Sinne dieser Erfindung stellt ein chimärer Promotor die Kombination einer stromaufwärts gelegenen zellspezifisch, metabolisch oder virusspezifisch aktivierbaren Aktivatorsequenz mit einem stromabwärts gelegenen Promotormodul dar. Das Promotormodul ist gekennzeichnet durch eine Nukleotidsequenz, welche die Transkriptionsfaktoren der Familien CDF und CHF oder E2F und CHF binden und hierdurch die Aktivierung der stromaufwärts gelegenen Aktivatorsequenz in der G0- und G1-Phase des Zellzyklus hemmen kann (Lucibello et al., EMBO J. 14, 132 (1994), PCT/GB95/02000).

In einer weiteren bevorzugten Ausführungsform ist bei den erfindungsgemäßen Nukleinsäurekonstrukten mindestens eine Promotor- oder Enhancersequenz (Komponente a) oder d)) eine Aktivator-responsive Promotoreinheit.

Eine Aktivator-responsive Promotoreinheit besteht ihrerseits aus folgenden Komponenten:
f) eine oder mehrere gleiche oder unterschiedliche Promotor- oder Enhancersequenz(en), die beispielsweise zellzyklus-spezifisch, metabolisch, zellspezifisch oder virusspezifisch oder sowohl zellzyklusspezifisch als auch metabolisch, zellspezifisch oder virusspezifisch (sogenannte chimäre Promotoren) aktivierbar ist bzw. sind,
g) eine oder mehrere gleiche oder unterschiedliche Aktivatorsubeinheit(en), welche jeweils stromabwärts von den Promotor- oder Enhancersequenzen gelegen und durch diese in ihrer basalen Transkription aktiviert wird bzw. werden, und
h) einen Aktivator-responsiven Promotor, welcher durch die Expressionsprodukte von einer oder mehreren Aktivatorsubeinheit(en) aktiviert wird.

Die Anordnung der einzelnen Komponenten einer bevorzugten Aktivator-responsiven Promotoreinheit ist durch Figur 3 wiedergegeben.

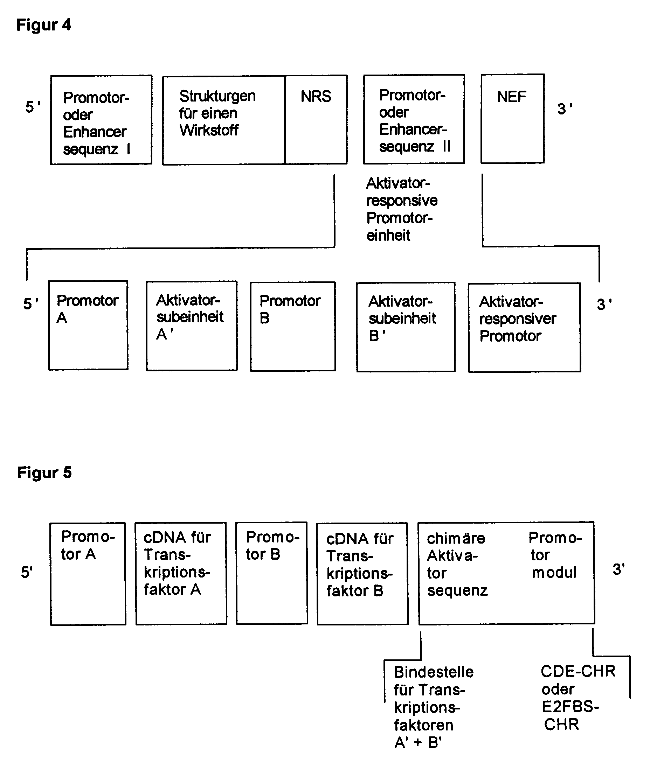

Die Einfügung einer bevorzugten Aktivator-responsiven Promotoreinheit in ein erfindungsgemäßes Nukleinsäurekonstrukt ist beispielsweise durch Figur 4 wiedergegeben.

In der einfachsten Form können Aktivator-responsive Promotoreinheiten beispielsweise chimäre Promotorkonstrukte gemäß Schema von Figur 5 darstellen.

In einer weiteren Ausführungsform können erfindungsgemäße Aktivator-responsive Promotoreinheiten Bindesequenzen für chimäre Transkriptionsfaktoren aus DNA-Bindungsdomänen, Protein-Proteininteraktionsdomänen und Transaktivierungsdomä-nen darstellen.

Bevorzugte Strukturgene für einen pharmakologisch aktiven Wirkstoff sind Gene für Ribozyme, Ribozyme mit kombinierter Antisense RNA, Proteine und Glykoproteine, ausgewählt aus der Gruppe umfassend Cytokine, Wachstumsfaktoren, Rezeptoren für Cytokine oder Wachstumsfaktoren, Fusionsproteine aus Liganden (z.B. Antikörper oder Antikörperfragmenten) und Cytokinen oder Wachstumsfaktoren antiproliferativ oder zytostatisch wirkende Proteine, Angiogeneseinhibitoren, Thrombose induzierende Proteine, Gerinnungshemmer, Komplement aktivierende Proteine, Hüllsubstanzen von Viren und Hüllsubstanzen von Bakterien.

Besonders bevorzugt sind Gene für Ribozyme, die spezifisch die mRNA von solchen Genen spalten, welche für Proteine kodieren, die an der Kontrolle des Zellzyklus im besonderen Maße beteiligt sind.

Zu diesen Proteinen gehören im besonderen Cyclin A, Cyclin D1, Cyclin E, Cyclin B, cdc2, E2F1-5, DP1, cdc25C (La Thangue, Current Opin. Cell Biol. 6, 443 (1994); Müller, Trends Genet. 11, 173 (1995); Zwicker et al., EMBO J. 14, 4514 (1995)).

Bei einer bevorzugten Ausführungsform ist das nukleare Retentions-Signal (NRS) eine Nukleotidsequenz, die den Transport einer mit ihr verknüpften premessenger RNA durch die Kernmembran behindert, die jedoch andererseits eine Bindestruktur darstellt für ein Exportprotein. Dieses Exportprotein vermittelt den Transport der ein NRS enthaltende premessenger oder messenger RNA aus dem Zellkern in das Zytoplasma. Eine, das NRS enthaltende premessenger oder messenger RNA wird somit durch Bindung an das Exportprotein aus dem Zellkern ausgeschleust (Fischer et al., Cell 82, 475 (1995)).

Bei den NRS handelt es sich bevorzugterweise um die Rev-Responsive Element (RRE)-Sequenz von Retroviren. Bei HIV-1 ist diese RRE eine 243 Nukleotide (Nukleotide 7362-7595; Muesing et al., Nature 313, 450 (1985)) umfassende Sequenz im env-Gen (Malim et al., Nature 338, 254 (1989); Kjems et al., PNAS 88, 683 (1991)). Das nukleare Retentions-Signal (NRS) im Sinne der Erfindung kann jedoch auch eine homologe und/oder funktionell ähnliche (analoge) Nukleotidsequenz sein, so beispielsweise das RRE-equivalente Element des HBV-Virus (Huang et al., Mol Cell Biol. 13, 7476 (1993)).

Bei den erfindungsgemäßen Nukleinsäurekonstrukten ist der nukleare Export-Faktor (NEF) eine Nukleotidsequenz, die für ein Protein kodiert, welches an die mRNA des NRS bindet und den Transport der ein NRS enthaltende premessenger RNA oder messenger RNA aus dem Zellkern in das Zytoplasma (oder aus dem Zytoplasma in den Zellkern) vermittelt. Im besonderen wird im Sinne der Erfindung das rev-Gen von Retroviren, speziell vom HIV-1 oder HIV-2 Virus (Daly et al., Nature 342, 816 (1989); Emerman et al., Cell 57, 1155 (1989); Felber et al., PNAS 86, 1495 (1989); Fischer et al., EMBO J. 13, 4105 (1994)) verwendet.

Das rev-Protein des rev-Genes von Retroviren bindet mit seiner N-terminalen Domäne (Zapp et al., Nature 342, 714 (1989); Malim et al., Cell 65, 241 (1991)) an das RRE in der pre-mRNA (Iwai et al., Nucl. Acids Res. 20, 6465 (1992)). Durch die Bindung zwischen dem RRE und dem rev-Protein wird der Transport von "nonspliced'' premessenger RNA, aber auch jeder weiteren RNA, die ein RRE enthält, vom Zellkern in das Zytoplasma ermöglicht (Fischer et al., EMBO J. 13, 4105 (1994); Fischer et al., Cell 82, 475 (1995)) und damit die Translation erheblich verstärkt.

Im Sinne der Erfindung können als NEF auch Nukleotidsequenzen verwendet werden, die Proteine kodieren, die homolog und funktionell ähnlich dem rev-Protein von HIV-1 sind (Bogerd et al., Cell 82, 485 (1995)), wie beispielsweise das rev-Gen des Visna-Maedi Virus (VMV; Tiley et al., J. Virol. 65, 3877 (1991)) oder das rev-Gen des Caprine arthritis encephalitis Virus (CAEV; Tiley et al., J. Virol. 65, 3877 (1991)).

Im Sinne der Erfindung können auch solche Gene eingesetzt werden, die Proteine kodieren, die zwar nur eine geringe oder keine Homologie zum rev-Protein besitzen, jedoch funktionell ähnlich dem rev-Protein von HIV-1 sind.

Hierzu zählen z.B. das rex-Gen des HTLV-1 (Cullen, Microbiol. Rev. 56, 375 (1992)), das rev-Gen des Virus der infektiösen Anämie des Pferdes (EIAV) und des Immundefizienzvirus der Katze (FIV) (Manusco et al., J. Virol. 68, 1988 (1994)).

In einer alternativen Ausführungsform kann es sich bei den NEF auch um Nukleotidsequenzen für Proteine handeln, welche eine Ausschleusung von RNA aus dem Kern bewirken, auch ohne daß diese durch ein NRS im Kern zurückgehalten wird. Hierzu zählen beispielsweise der Transkriptionsfaktor TFIIIA (Gaddat et al., Cell 60, 619 (1990)) oder das heterogene nukleäre Ribonukleoprotein A1 (hnRNPA1-Protein; Pinol-Roma et al., Nature 355, 730 (1992)).

Des weiteren gehören zu den nukleären Transportproteinen im erweiterten Sinne das "heat shock protein 70" (hsc70; Mandell et al., J. Cell Biol. 111, 1775 (1990)) oder der Pro▼teinkinaseinhibitor CPKI (Fantozzi et al., J. Biol. Chem. 269, 2676 (1994), Wen et al., J. Biol. Chem. 269, 32214 (1994)).

Gemeinsam ist dem NEF und seinen homologen und analogen Proteinen eine mehr aminoterminal gelegene Domäne für die Bindung des monomeren Proteins an die RNA des NRS (J. Virol 64, 881 (1990); Kjems et al., EMBO J. 11, 1119 (1992)) und eine meist Leucin-reiche Domäne (Ausnahme hiervon hnRNPA1), welche für die Transportfunktion des NEF notwendig ist (Wen et al., Cell 82, 463 (1995); Fischer et al., Cell 82, 475 (1995); Malim et al., J. Virol. 65, 4248 (1991); Venkatesh et al. Virol. 178, 327 (1990)).

Die Nukleinsäurekonstrukte bestehen bevorzugterweise aus DNS. Unter dem Begriff Nukleinsäurekonstrukte werden künstliche Gebilde aus Nukleinsäure verstanden, die in den Zielzellen transkribiert werden können. Sie sind bevorzugt in einen Vektor eingefügt, wobei Plasmidvektoren oder virale Vektoren besonders bevorzugt sind.

Durch die erfindungsgemäßen Nukleinsäurekonstrukte kann ein Transgen (Komponente b) sowohl zellspezifisch oder virusspezifisch oder unter bestimmten metabolischen Bedingungen als auch zellzyklusspezifisch exprimiert werden, wobei es sich bei dem Strukturgen bevorzugt um ein Gen handelt, das für einen pharmakologisch aktiven Wirkstoff oder aber für ein Enzym kodiert, welches eine inaktive Vorstufe eines Pharmakons in ein aktives Pharmakon spaltet. Das Strukturgen kann so gewählt sein, daß dieses Enzym als Fusionsprotein mit einem Liganden exprimiert wird und dieser Ligand an die Oberfläche von Zellen, z.B. proliferierende Endothelzellen oder Tumorzellen bindet.

Gegenstand der vorliegenden Erfindung sind auch Zellen von Hefen oder Säugern, die ein erfindungsgemäßes Nukleinsäurekonstrukt enthalten. In besonders bevorzugter Ausführungsform werden die Nukleinsäurekonstrukte in Zellinien eingebracht, die dann nach Transfektion zur Expression des Transgens verwendet werden können. Diese Zellen können somit zur Bereitstellung eines Heilmittels für Patienten wie auch im Patienten und somit zur Behandlung einer Erkrankung verwendet werden. Eine bevorzugte Verwendung des erfindungsgemäßen Nukleinsäurekonstruktes besteht in der Behandlung einer Erkrankung, wobei die Bereitstellung des Heilmittels die Einführung eines Nukleinsäurekonstrukts in eine Zielzelle und dessen virus- oder zielzellspezifische und zellzyklusspezifische Expression umfaßt. Bei der Erkrankung handelt es sich häufig um eine solche, die mit übermäßiger Zellvermehrung einhergeht, wobei die Herstellung des Heilmittels die Einführung eines Nukleinsäurekonstruktes in eine Zielzelle und seine virus- oder zielzellspezifische Expression in dem Stadium der Zellproliferation umfaßt.

Die erfindungsgemäßen Nukleinsäurekonstrukte kommen in dieser Form nicht in der Natur vor, d.h. das Transgen oder Strukturgen für den Wirkstoff oder für ein Enzym oder für ein Ligand-Enzym-Fusionsprotein ist nicht natürlicherweise kombiniert mit dem nuklearen Retentions-Signal (NRS) und beide sind nicht natürlicherweise mit dem Promotor I verbunden und diese Kombination ist wiederum nicht natürlicherweise kombiniert mit der Nukleotidsequenz bestehend aus dem Promotor II und dem nuklearen Export-Faktor (NEF).

Die Promotoren I und II und das Strukturgen für den Wirkstoff (oder für das Enzym) der erfindungsgemäßen Nukleinsäurekonstrukte werden je nach Verwendungszweck ausgewählt.

Nachfolgend werden einige bevorzugte Anwendungsmöglichkeiten dargestellt:

### 1.1 Kombination eines starken unspezifischen Promotors oder Enhancers mit einem chimären Promotor für die Expression eines Strukturgenes

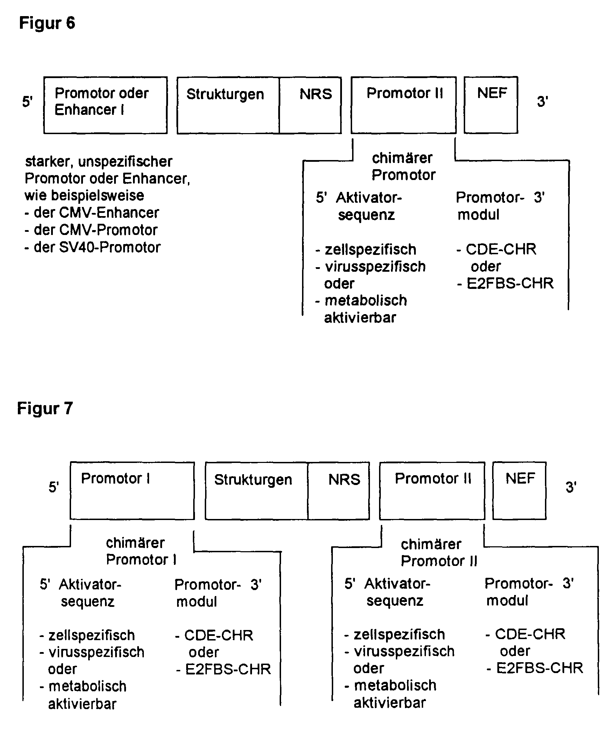

Beispiele für solche Kombinationen sind in Figur 6 dargestellt.

### 1.2 Kombination zweier unterschiedlicher oder gleicher chimärer Promotoren für die Expression eines Strukturgenes

Beispiele für solche Kombinationen sind in Figur 7 dargestellt.

### 1.3 Kombination zweier unterschiedlicher oder gleicher Promotoren oder Enhancer für die Expression eines Strukturgenes

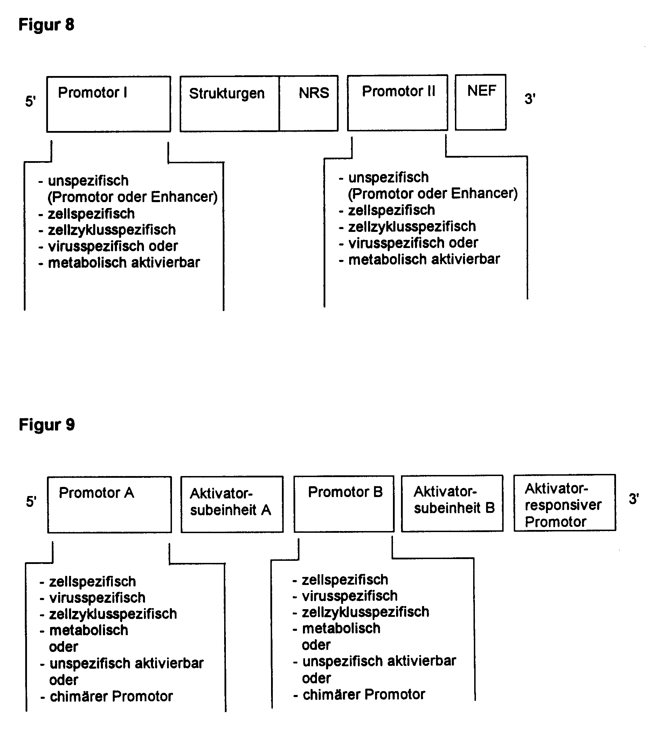

Beispiele für solche Kombinationen sind in Figur 8 dargestellt.

### 1.4 Aktivator-responsiver Promotor

Beispiele für solche Kombinationen sind in Figur 9 dargestellt.

### 1.5 Mehrfachkombination von unterschiedlichen Promotoren zur mehrfach kontrollierten Expression eines Strukturgenes

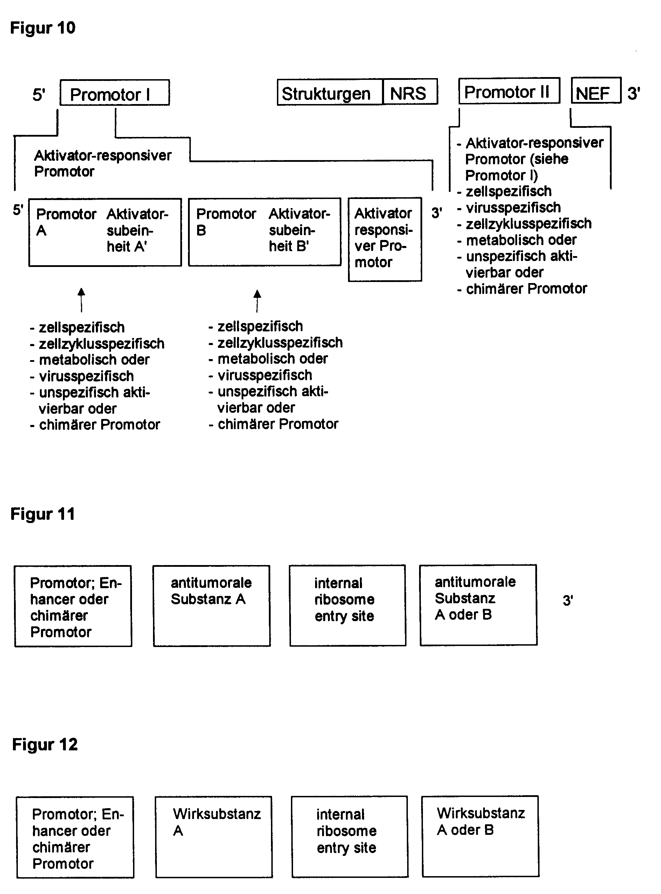

Beispiele für solche Kombinationen sind in Figur 10 dargestellt.

In Abhängigkeit von dem geplanten Einsatz der Nukleinsäure-Konstrukte können folgende Ausführungsformen ausgewählt werden:

### 2. Therapie von Tumoren und chronischen Entzündungen über die Inhibition des proliferierenden Endothels

### 2.1.a) Auswahl der Promotoren oder Aktivatorsequenzen aktiviert in Endothelzellen

Im Sinne dieser Erfindung zählen zu den bevorzugten Promotoren oder Aktivatorsequenzen aus Promotoren oder Enhancern solche genregulatorische Sequenzen bzw. Elemente für Gene, die für besonders in Endothelzellen (oder aber in Zellen in unmittelbarer Nachbarschaft mit proliferierenden Endothelzellen) nachweisbare Proteine kodieren.

Einige dieser Proteine sind von Borrows et al. (Pharmac. Ther. 64, 155 (1994)) und Pate et al. (Brain Pathol. 4, 207 (1994)) beschrieben worden. Im besonderen zählen zu diesen Endothelzell-spezifischen Proteinen beispielsweise:
- Hirn-spezifischer, endothelialer Glucose-1-Transporter
   Die Promotorsequenz wurde von Murakami et al. (J. Biol. Chem. 267, 9300 (1992)) beschrieben.
- Endoglin
   Die Promotorsequenz wurde von Bellon et al. (Eur. J., Immunol. 23, 2340 (1993)) und Ge et al. (Gene 138, 201 (1994)) beschrieben.
- VEGF-Rezeptoren
   Zwei Rezeptoren werden unterschieden (Plate et al., Int. J. Cancer 59, 520 (1994)):
   - VEGF-Rezeptor-1 (flt-1)
      (de Vries et al., Science 255, 989 (1992)) und der
   - VEGF-Rezeptor-2 (flk-1, KDR)
      (Terman et al., BBRC 187, 1579 (1992)).

Beide Rezeptoren sind fast ausschließlich auf endothelialen Zellen (Senger et al., Cancer Metast. Rev. 12, 303 (1993)) anzutreffen.
- andere Endothelzell-spezifische Rezeptortyrosinkinasen
   - til-1 oder til-2
      (Partanen et al., Mol. Cell. Biol. 12, 1698 (1992), Schnürch und Risau, Development 119, 957 (1993), Dumont et al., Oncogene 7, 1471 (1992))
   - B61-Rezeptor (Eck-Rezeptor)
      (Bartley et al., Nature 368, 558 (1994), Pandey et al., Science 268, 567 (1995), van der Geer et al., Ann. Rev. Cell. Biol. 10, 251 (1994))
- B61
   Das B61-Molekül stellt den Liganden dar für den B61-Rezeptor.
   (Holzman et al., J. Am. Soc. Nephrol. 4, 466 (1993), Bartley et al., Nature 368, 558 (1994))
- Endothelin, im speziellen
   - Endothelin B
      Die Promotorsequenz wurde von Benatti et al., J. Clin. Invest. 91, 1149 (1993) beschrieben.
   - Endothelin-1
      Die Promotorsequenz wurde von Wilson et al., Mol. Cell. Biol. 10, 4654 (1990) beschrieben.
- Endothelin-Rezeptoren, insbesondere der Endothelin B-Rezeptor
   (Webb et al., Mol. Pharmacol. 47, 730 (1995), Haendler et al. J. Cardiovasc. Pharm. 20, 1 (1992)).
- Mannose-6-Phosphat-Rezeptoren
   Die Promotorsequenzen sind von Ludwig et al. (Gene 142, 311 (19949), Oshima et al., (J. Biol. Chem. 263, 2553 (1988)) und Pohlmann et al. (PNAS USA 84, 5575 (1987)) beschrieben worden.
- Von Willebrand Faktor
   Die Promotorsequenz wurde von Jahroudi und Lynch (Mol. Cell. Biol. 14, 999 (1994)), Ferreira et al. (Biochem. J. 293, 641 (1993)) und Aird et al. (PNAS USA 92, 4567 (1995)) beschrieben.
- IL-1α, IL-1β
   Die Promotorsequenzen wurden von Hangen et al., Mol. Carcinog. 2, 68 (1986), Turner et al., J. Immunol. 143, 3556 (1989), Fenton et al., J. Immunol. 138, 3972 (1987), Bensi et al., Cell Growth Diff. 1, 491 (1990), Hiscott et al., Mol. Cell. Biol. 13, 6231 (1993) und Mori et al., Blood 84, 1688 (1994) beschrieben.
- IL-1-Rezeptor
   Die Promotorsequenz wurde von Ye et al., PNAS USA 90, 2295 (1993) beschrieben.
- Vascular Cell Adhesion Molecule (VCAM-1)
   Die Promotorsequenz des VCAM-1 wurde beschrieben von Neish et al., Mol. Cell. Biol. 15, 2558 (1995), Ahmad et al., J. Biol. Chem. 270, 8976 (1995), Neish et al., J. Exp. Med. 176, 1583 (1992), Iademarco et al., J. Biol. Chem. 267, 16323 (1992), und Cybulsky et al., PNAS USA 88, 7859 (1991)
- Synthetische Aktivatorsequenz
   Als Alternative zu natürlichen endothelspezifischen Promotoren lassen sich auch synthetische Aktivatorsequenzen verwenden, die aus oligomerisierten Bindestellen für Transkriptionsfaktoren, die preferentiell oder selektiv in Endothelzellen aktiv sind, bestehen. Ein Beispiel hierfür ist der Transkriptionsfaktor GATA-2, dessen Bindestelle im Endothelin-1 Gen 5'-TTATCT-3' ist (Lee et al., Biol. Chem. 266, 16188 (1991), Dorfmann et al., J. Biol. Chem. 267, 1279 (1992) und Wilson et al., Mol. Cell Biol. 10, 4854 (1990)).

### 2.1.b) Auswahl der Promotoren oder Aktivatorsequenzen, aktiviert in Zellen in Nachbarschaft aktivierter Endothelzellen

Bei proliferierenden Endothelien werden durch geöffnete "tight junctions" Nachbarzellen für Makromoleküle vom Blut aus zugänglich. Durch die funktionellen und anatomischen Wechselbeziehungen sind die Nachbarzellen von aktivierten Endothelzellen Zielzellen im Sinne dieser Erfindung.
- VEGF
   Die genregulatorischen Sequenzen für das VEGF-Gen sind
   - die Promotorsequenz des VEGF-Gens (5' flankierende Region)
      (Michenko et al., Cell. Mol. Biol. Res. 40, 35 (1994), Tischer et al., J. Biol. Chem. 266, 11947 (1991)) oder
   - die Enhancersequenz des VEGF-Gens (3' flankierende Region)
      (Michenko et al., Cell Mol. Biol. Res. 40, 35 (1994)) oder
   - das c-Src-Gen
      (Mukhopadhyay et al., Nature 375, 577 (1995), Bonham et al., Oncogene 8, 1973 (1993), Parker et al., Mol. Cell. Biol. 5, 831 (1985), Anderson et al., Mol. Cell. Biol. 5, 112 (1985)) oder
   - das V-Scr-Gen
      (Mukhodpadhyay et al., Nature 375, 577 (1995), Anderson et al., Mol. Cell. Biol. 5, 112 (1985), Gibbs et al., J. Virol. 53, 19 (1985))
- Steroid-Hormonrezeptoren und deren Promotorelemente (Truss und Beato, Endocr. Rev. 14, 459 (1993)), insbesondere der
   - Maus-Mammatumor-Virus-Promotor
      Die cDNA-Sequenz der Promotorregion der long terminal repeat region des MMTV wurde von Chalepakis et al., Cell 53, 371 (1988) und Truss und Beato (Endocr. Rev. 14, 459 (1993) beschrieben.

### 2.2. Strukturgene für antitumorale (oder antientzündliche) Substanzen

### 2.2.a) Inhibitoren der Proliferation

Als antitumorale oder antientzündliche Substanz im Sinne dieser Erfindung ist die DNA-Sequenz eines Proteins zu verstehen, welches die Proliferation von Endothelzellen inhibiert. Hierzu gehören beispielsweise die DNA-Sequenzen für:
- das Retinoblastomprotein (pRb/p110) oder für seine Analoga p107 und 120
- das p53 Protein
- das p21 (WAF-1) Protein
- das p16 Protein
- weitere CdK-Inhibitoren
- das GADD45 Protein
- das bak Protein.

Um eine schnelle intrazelluläre Inaktivierung dieser Zellzyklusinhibitoren zu verhindern, sind bevorzugt solche Gene zu verwenden, welche Mutationen für die ♂ Inaktivierungsstellen der exprimierten Proteine aufweisen, ohne daß diese hierdurch in ihrer Funktion beeinträchtigt werden.

Das Retinoblastomprotein (pRb) und die verwandten p107 und p130 Proteine werden durch Phosphorylierung inaktiviert. Bevorzugt wird somit eine pRb/p110 -, p107 - oder p130 cDNA-Sequenz verwendet, die derartig punktmutiert ist, daß die Phosphorylierungsstellen des kodierten Proteins gegen nicht phosphorylierbare Aminosäuren ausgetauscht sind.

### 2.2.b) Gerinnung induzierende Faktoren und Angiogeneseinhibitoren

Als antitumorale bzw. antientzündliche Substanz ist des weiteren die DNA-Sequenz für ein Protein zu verstehen, welches die Gerinnung induziert und/oder die Angiogenese inhibiert. Zu diesen Proteinen zählt beispielsweise:
- Tissue factor (TF) und gerinnungsaktive Fragmente von ihm (Morrissey et al., Cell 50, 129 (1987), Scarpati et al., Biochem. 26, 5234 (1987), Spicer et al., PNAS USA 84, 5148 (1987), Rehemtulla et al., Thromb. Heamost. 65, 521 (1991))
- Plasminogenaktivatorinhibitor-1 (PAI-1)
- PAI-2
- PAI-3
- Angiostatin
- Interferone
   - IFNα
   - IFNβ
   - IFNγ
- Platelet factor 4
- IL-12
- TIMP-1
- TIMP-2
- TIMP-3
- Leukemia Inhibitory Factor (LIF).

### 2.2.c) zytostatische und zytotoxische Proteine

Als antitumorale bzw. antientzündliche Substanz ist jedoch auch eine DNA-Sequenz für ein Protein zu verstehen, welches direkt oder indirekt eine zytostatische Wirkung auf Tumoren aufweist. Hierzu zählen im besonderen:
- Antikörper oder Antikörperfragmente
- Perforin
- Granzym
- IL-2
- IL-4
- IL-12
- Interferone, wie beispielsweise
   - IFNα
   - IFNβ
   - IFNγ
- TNF
   - TNFα
   - TNFβ
- Oncostatin M
- Sphingomyelinase (Jarvis et al. PNAS - USA 91, 73, (1994))
- Magainin und Magaininderivate (Cruciani et al. PNAS 88, 3792 (1991); Jacob et al. Ciba Found, Symp. 186, 197 (1994); Peck-Miller et al. Cancer Chemoth., Pharmac. 32, 109 (1993))

### 2.2.d) Induktoren von Entzündungen

Als antitumorale Substanz ist des weiteren die DNA-Sequenz für ein Protein zu verstehen, welches ggf. zusätzlich zur antitumoralen Wirkung Entzündungen stimuliert und hierdurch zur Elimination von Tumorzellen beiträgt. Hierzu zählen im besonderen beispielsweise:
- RANTES (MCP-2)
- monocyte chemotactic and activating factor (MCAF)
- IL-8
- macrophage inflammatory protein-1 (MIP-1α, -β)
- neutrophil activating protein-2 (NAP-2)
- IL-3
- IL-5
- human leukemia inhibitory factor (LIF)
- IL-7
- IL-11
- IL-13
- GM-CSF
- G-CSF
- M-CSF
- Cobra venom factor (CVF) oder Teilsequenzen vom CVF, welche dem menschlichen Komplementfaktor C3b funktionell entsprechen, d.h. welche an den Komplementfaktor B binden können und nach Spaltung durch den Faktor D eine C3 Konvertase darstellen. Die DNA-Sequenz für CVF und seiner Teilsequenzen wurden von Fritzinger et al., Proc.Natl.Acad.Sci. USA 91, 12775 (1994) veröffentlicht.
- der menschliche Komplementfaktor C3 oder seine Teilsequenz C3b. Die DNA-Sequenz für C3 und seiner Teilsequenzen wurde von De Bruijn et al., Proc.Natl.Acad.Sci. USA 82, 708 (1985) publiziert.
- Spaltprodukte des menschlichen Komplementfaktors C3, welche funktionell und strukturell dem CVF ähneln. Derartige Spaltprodukte wurden von O'Keefe et al., J.Biol.Chem. 263, 12690 (1988) beschrieben.
- Bakterielle Proteine, welche Komplement aktivieren oder Entzündungen auslösen, wie beispielsweise Porine von Salmonella typhimurium (Galdiero et al., Infection and Immunity 46, 559 (1984)), "clumping" Faktoren von Staphylococcus aureus (Espersen Acta Path. Microb. et Imm. Scandin. Sect C 93, 59 (1985)), Moduline besonders von gram-negativen Bakterien (Henderson et al., Inflam.Res. 44, 187 (1995)), "Major outer membrane protein" von Legionellen (Bellinger-Kawahara et al., J.Exp.Med. 172, 1201 (1990)) oder von Haemophilus influenzae Typ B (Hetherington et al. Infection and Immunity 60, 19 (1992)) oder von Klebsiellen (Alberti et al., Infection and Immunity 61, 852 (1993)) oder M-Moleküle von Streptokokken Gruppe G (Campo et al., J.Infec.Dis. 171, 601 (1995)).

Als Wirksubstanz im Sinne der Erfindung können auch DNA-Sequenzen von Fusionsproteinen zwischen den aufgeführten Cytokinen oder Wachstumsfaktoren zum einen und Liganden für Rezeptoren auf der Zellmembran (wie beispielsweise einem Antikörper spezifisch für Endothelzellen oder Tumorzellen oder dem Fc-Teil des menschlichen Immunglobulins) zum anderen Verwendung finden. Derartige DNA-Sequenzen und ihre Herstellung wurden beispielsweise in der EPA 0464 633 A1 beschrieben.

### 2.2.e) Enzyme für die Aktivierung von Vorstufen von Zytostatika

Als antitumorale bzw. antientzündliche Substanz ist jedoch auch die DNA-Sequenz für ein Enzym zu verstehen, welches in der Lage ist, Vorstufen eines antitumoralen Wirkstoffes in einen antitumoralen Wirkstoff zu überführen.

Derartige Enzyme, welche inaktive Vorsubstanzen (Prodrugs) in aktive Zytostatika (Drugs) spalten und die jeweils zugehörigen Prodrugs und Drugs sind bereits von Deonarain et al. (Br. J. Cancer 70, 786 (1994)), von Mullen, Pharmac. Ther. 63, 199 (1994)) und Harris et al. (Gene Ther. 1, 170 (1994)) übersichtlich beschrieben worden.

Beispielsweise ist die DNA-Sequenz eines der folgenden Enzyme zu verwenden:
- Herpes Simplex Virus thymidinkinase
   (Garapin et al., PNAS USA 76, 3755 (1979), Vile et al., Cancer Res. 53, 3860 (1993), Wagner et al., PNAS USA 78, 1441 (1981), Moelten et al., Cancer Res. 46, 5276 (1986), J. Natl. Cancer Inst. 82, 297 (1990))
- Varizella Zoster Virus Thymidinkinase
   (Huber et al., PNAS USA 88, 8039 (1991), Snoeck, Int. J. Antimicrob. Agents 4, 211 (1994))
- bakterielle Nitroreduktase
   (Michael et al., FEMS Microbiol. Letters 125, 195 (1994), Bryant et al., J. Biol. Chem. 266, 4126 (1991), Watanabe et al., Nucleic Acids Res. 18, 1059 (1990))
- bakterielle β-Glucuronidase
   (Jefferson et al., PNAS USA 83, 8447 (1986))
- pflanzliche β-Glucuronidase aus Secale cereale
   (Schulz et al., Phytochemistry 26, 933 (1987))
- humane β-Glucuronidase
   (Bosslet et al., Br. J. Cancer 65, 234 (1992), Oshima et al., PNAS USA 84, 685 (1987))
- humane Carboxy peptidase (CB) z.B.
   - CB-A der Mastzelle
      (Reynolds et al., J. Clin. Invest. 89, 273 (1992))
   - CB-B des Pankreas
      (Yamamoto et al., J. Biol. Chem. 267, 2575 (1992), Catasus et al., J. Biol. Chem. 270, 6651 (1995))
   - bakterielle Carboxy peptidase
      (Hamilton et al., J. Bacteriol. 174, 1626 (1992), Osterman et al., J. Protein Chem. 11, 561 (1992))
- bakterielle β-Laktamase
   (Rodrigues et al., Cancer Res. 55, 63 (1995), Hussain et al., J. Bacteriol. 164, 223 (1985), Coque et al., EMBO J. 12, 631 (1993))
- bakterielle Cytosine deaminase
   (Mullen et al., PNAS USA 89, 33 (1992), Austin et al., Mol. Pharmac. 43, 380 (1993), Danielson et al., Mol. Microbiol. 6, 1335 (1992))
- humane Catalase bzw. Peroxidase
   (Ezurum et al., Nucl. Acids Res. 21, 1607 (1993))
- Phosphatase, im besonderen
   - humane alkalische Phosphatase
      (Gum et al., Cancer Res. 50, 1085 (1990))
   - humane saure Prostataphosphatase
      (Sharieff et al., Am. J. Hum. Gen. 49, 412 (1991), Song et al., Gene 129, 291 (1993), Tailor et al., Nucl. Acids Res. 18, 4928 (1990))
   - Typ 5 saure Phosphatase
   (Gene 130, 201 (1993))
- Oxidase, im besonderen
   - humane Lysyloxidase
      (Kimi et al., J. Biol. Chem. 270, 7176 (1995))
   - humane saure D-aminooxidase (Fukui ei al., J. Biol. Chem. 267, 18631 (1992))
- Peroxidase, im besonderen
   - humane Glutathion Peroxidase
      (Chada et al., Genomics 6, 268 (1990), Ishida et al., Nucl. Acids Res. 15, 10051 (1987))
   - humane Eosinophilen Peroxidase (Ten et al., J. Exp. Med. 169, 1757 (1989), Sahamaki et al., J. Biol. Chem. 264, 16828 (1989))
   - humane Schilddrüsen Peroxidase (Kimura, PNAS USA 84, 5555 (1987)).
- Galactosidasen

Zur Erleichterung der Sekretion der aufgeführten Enzyme kann die jeweils in der DNA-Sequenz enthaltende homologe Signalsequenz ersetzt werden durch eine heterologe, die extrazelluläre Ausschleusung verbessernde Signalsequenz.

So kann beispielsweise die Signalsequenz der β-Glucuronidase (DNA Position ≤ 27 bis 93; Oshima et al., PNAS 84, 685 (1987)) ersetzt werden durch die Signalsequenz für das Immunglobulin (DNA Position ≤ 63 bis ≥ 107; Riechmann et al., Nature 332, 323 (1988)) oder durch die Signalsequenz für das CEA (DNA-Position ≤ 33 bis ≥ 134; Schrewe et al., Mol. Cell. Biol. 10, 2738 (1990), Berling et al., Cancer Res. 50, 6534 (1990)) oder durch die Signalsequenz des humanen respiratory syncytial virus glycoproteins (cDNA der Aminosäuren ≤ 38 bis ≥ 50 oder 48 bis 65; Lichtenstein et al., J. General Virol. 77, 109 (1996)).

Des weiteren sind bevorzugt DNAs solcher Enzyme zu wählen, welche durch Punktmutation in einem geringeren Maße in Lysosomen gespeichert und vermehrt sekretiert werden. Derartige Punktmutationen wurden beispielsweise für die β-Glucuronidase beschrieben (Shiplex et al., J.Biol.Chem. 268, 12193 (1993).

Zur Verankerung des Enzyms in die Zellmembran der das Enzym bildenden Zelle kann alternativ oder zusätzlich zur Signalsequenz eine Sequenz für eine Transmembrandomäne eingeführt werden.

So kann beispielsweise die Transmembransequenz des menschlichen Makrophagenkolonie-stimulierenden Faktors (DNA-Position ≤ 1485 bis ≥ 1554; Cosman et al., Behring Inst. Mitt. 83, 15 (1988)) oder die DNA-Sequenz für die Signal- und Transmembranregion des menschlichen Respirator Syncytial Virus (RSV)-Glykoproteins G (Aminosäuren 1 bis 63 oder deren Teilsequenzen, Aminosäuren 38 bis 63; Vijaya et al., Mol. Cell Biol. 8, 1709 (1988), Lichtenstein et al., J. General Virol. 77, 109 (1996)) oder die DNA-Sequenz für die Signal- und Transmembranregion der Influenzavirus-Neuraminidase (Aminosäuren 7 bis 35 oder die Teilsequenz Aminosäuren 7 bis 27; Brown et al., J. Virol. 62, 3824 (1988)) zwischen der DNA-Sequenz für den Promotor und der DNA-Sequenz für das Enzym (z.B. die β-Glucuronidase) eingefügt werden.

Zur Verstärkung der Translation kann am 3'-Ende des Promotors und unmittelbar vor dem 5'-Ende des Startsignals (ATG) der Signal- bzw. Transmembransequenz die Nukleotidsequenz
GCCACC
oder
GCCGCC
eingefügt (Kozak, J. Cell. Biol. 108, 299 (1989) werden.

Zur Verankerung des Enzyms in die Zellmembran der das Enzym bildenden Zellen kann jedoch auch die Nukleotidsequenz für einen Glykophospholipid-Anker eingefügt werden.

Die Einfügung eines Glykophospholipid-Ankers erfolgt am 3'-Ende der Nukleotidsequenz für das Enzym und kann zusätzlich zur Einfügung einer Signalsequenz erfolgen.

Glykophospholipid-Anker sind beispielsweise für das CEA (DNA-Position ≤ 893 bis ≥ 1079; Berling et al., Cancer Res. 50, 6534 (1990)), für das N-CAM (Cunningham et al., Science 236, 799 (1987) und für weitere Membranproteine, wie beispielsweise Thy-1 (Clissold, Biochem. J. 281, 129 (1992)) oder CD16 (Selvaray et al., Nature 333, 565 (1988)) beschrieben worden.

Eine Übersicht über Glycophospholipid verankerte Membranproteine wurde von Ferguson et al. (Ann. Rev. Biochem. 57, 285 (1988)) publiziert.

Eine weitere Möglichkeit der Verankerung von Enzymen an die Zellmembran entsprechend der vorliegenden Erfindung ist die Verwendung einer DNA-Sequenz für ein Ligand-Enzym-Fusionsprotein. Die Spezifität des Liganden dieses Fusionsproteins ist gerichtet gegen ein auf der Zellmembran von proliferierenden Endothelzellen oder von Tumorzellen befindliche Membranstruktur.

Zu den Liganden, welche an die Oberfläche von proliferierenden Endothelzellen binden, gehören beispielsweise Antikörper oder Antikörperfragmente, gerichtet gegen Membranstrukturen von Endothelzellen, wie sie beispielsweise von Burrows et al. (Pharmac.Ther. 64, 155 (1994)), Hughes et al. (Cancer Res. 49, 6214 (1989)) und Maruyama et al. (PNAS-USA 87, 5744 (1990)) beschrieben wurden. Insbesondere zählen hierzu Antikörper gegen die VEGF-Rezeptoren.

Die murinen monoklonalen Antikörper sind bevorzugt in humanisierter Form einzusetzen. Die Humanisierung erfolgt in der von Winter et al. (Nature 349, 293 (1991)) und Hoogenbooms et al. (Rev.Tr.Transfus.Hemobiol. 36, 19 (1993)) dargestellten Weise. Antikörperfragmente werden entsprechend dem Stand der Technik hergestellt, beispielsweise in der von Winter et al. (Nature 349, 293 (1991)), Hoogenboom et al. (Ref.Tr.Transfus.Hemobiol. 36, 19 (1993); Girol.Mol.Immunol. 28, 1379 (1991) oder Huston et al. (Intern.Rev.Immunol. 10, 195 (1993)) beschriebenen Weise.

Zu den Liganden gehören des weiteren alle Wirkstoffe, welche an Membranstrukturen oder Membranrezeptoren auf Endothelzellen binden. Beispielsweise gehören hierzu Substanzen, die endständig Mannose enthalten, des weiteren IL-1 oder Wachstumsfaktoren oder deren Fragmente bzw. Teilsequenzen von Ihnen, die an Rezeptoren exprimiert durch Endothelzellen binden, wie beispielsweise PDGF, bFGF, VEGF, TGFβ (Pusztain et al., J.Pathol. 169, 191 (1993)). Des weiteren gehören hierzu Adhäsionsmoleküle, welche an aktivierte und/oder proliferierende Endothelzellen binden. Derartige Adhäsionsmoleküle, wie beispielsweise SLex, LFA-1, MAC-1, LECAM-1 oder VLA-4, wurden bereits beschrieben (Übersichten bei Augustin-Voss et al., J.Cell.Biol. 119, 483 (1992), Pauli et al., Cancer Metast. Rev. 9, 175 (1990), Honn et al., Cancer Metast. Rev. 11, 353 (1992)).

Zu den Liganden gehören jedoch auch Antikörper oder deren Fragmente, welche gerichtet sind gegen tumorspezifische oder tumorassoziierte Antigene auf der Tumorzellmembran.

Beispiele für derartige Antigene und die zugehörigen Antikörper sind bei Sedlacek et al., Contrib.Oncol. 32 (1988) und Contrib.Oncol. 43 (1992) beschrieben. Antikörper-Enzym-Fusionsproteine wurden beispielsweise von Bosslet et al., Br. J. Cancer 65, 234 (1992) beschrieben. Zur Erleichterung der Sekretion der aufgeführten Ligand-Enzym-Fusionsproteine kann die jeweils in der DNA-Sequenz des Enzyms enthaltene homologe Signalsequenz, wie bereits beschrieben, ersetzt werden durch eine heterologe, die extrazelluläre Ausschleusung verbessernde Signalsequenz.

### 2.2.f) Ribozyme

Als antitumorale bzw. antientzündliche Substanz ist jedoch auch die DNA-Sequenz für ein Ribozym zu verstehen, welches in der Lage ist, das Transkriptionsprodukt (messenger RNA) eines Genes für ein Zellzykluskontrollprotein zu spalten und hierdurch zu inaktivieren.

Bevorzugte Substrate für derartige Ribozyme ist die messenger RNA der Gene des Cyclin A, Cyclin B, Cyclin D1, Cyclin E, cdc2, cdc25C und DP1.

Die DNA-Oligonukleotidsequenz für ein Ribozym, welches Cyclin D1 mRNA (Xiong et al., Cell 665, 691 (1991) in der Nukleotid-Position (301) CUG CC GUC C AUG (312) spaltet, ist beispielsweise
SEQ ID NO.: 1
   5'-AGCTTCCGCATGCTGATGAGGCCGCAAGGCCGAAACGGCAG-3'
SEQ ID NO.: 2
   5'-AATTCTGCCGTTTCGGCCTTGCGGCCTCATCAGCATGCGGA-3'

Die DNA-Oligonukleotidsequenz für ein weiteres Ribozym zur Spaltung der Cyclin D1 mRNA in der Nukleotidposition (406) UUC CU GUC G CUG (417) (Xiong et al., 1991) ist beispielsweise
SEQ ID NO.: 3
   5'-AGCTTCCAGCCTGATGAGGCCGCAAGGCCGAAACAGGAAG-3'
SEQ ID NO.: 4
   5'-AATTCTTCCTGTTTCGGCCTTGCGGCCTCATCAGGCTGGA-3'

Die DNA-Oligonukleotidsequenz für ein Ribozym, welches die mRNA von Cyclin E (Koff et al., Cell 66, 1217 (1991)) in der Nukleotid-Position (208) GCA GUC UGU (216) (Xiong et al., 1991) spaltet, ist beispielsweise
SEQ ID NO.: 5
   5'-AGCTTGCACACTGATGAGGCCGCAAGGCCGAAACTGCG-3'
SEQ ID NO.: 6
   5'-AATTCGCAGTTTCGGCCTTGCGGCCTCATCAGTGTGCA-3'
die DNA Oligonukleotidsequenz für ein weiteres Ribozym zur Spaltung der mRNA von Cyclin E in der Nukleotid-Position (481) GAA GUC UAU (489) (Xiong et al., 1991) spaltet, ist beispielsweise
SEQ ID NO.: 7
   5'-AGCTTGAAGTTTATACTGATGAGGCCGCAAGGCCGAAACTTCAG-3'
SEQ ID NO.: 8
   5'-AATTCTGAAGTTTCGGCCTTGCGGCCTCATCAGTATAAACTTCA-3'

### 2.3. Kombination von mehreren antitumoralen oder antientzündlichen Substanzen

Gegenstand der Erfindung sind des weiteren Nukleinsäurekonstrukte, in welchen eine Kombination der DNA-Sequenzen von mehreren gleichen antitumoralen oder antientzündlichen Substanzen (A,A) oder von unterschiedlichen antitumoralen Substanzen (A,B) vorliegt. Zur Expression von zwei DNA-Sequenzen wird vorzugsweise die cDNA einer "internal ribosome entry site" (IRES) als regulatorisches Element zwischengeschaltet. Dies wird durch Figur 11 illustriert.

Derartige IRES wurden beispielsweise von Mountford und Smith (TIG 11, 179 (1995), Kaufman et al., Nucl. Acids Res. 19, 4485 (1991), Morgan et al., Nucl. Acids Res. 20, 1293 (1992), Dirks et al., Gene 128, 247 (1993), Pelletier und Sonenberg, Nature 334, 320 (1988) und Sugitomo et al., BioTechn. 12, 694 (1994) beschrieben.

So kann die cDNA der IRES-Sequenz des Poliovirus (Position ♂ú 140 bis | 630 des 5' UTR; Pelletier und Sonenberg, Nature 334, 320 (1988)) zur Verknüpfung der DNA der antientzündlichen Substanz A (am 3' Ende) und der DNA der antientzündlichen Substanz B (am 5' Terminus) verwendet werden.

Ein derartiger Wirkstoff weist je nach Kombination additive (A+A, A+B1) oder synergistische Wirkung im Sinne der Erfindung auf.

### 3. Wirkstoff zur Behebung der mangelhaften Bildung von Zellen des Blutes

### 3.1. Auswahl der Promotoren oder der Aktivatorsequenzen für blutbildende Zellen

Im Sinn der vorliegenden Erfindung wird als Promotor oder Aktivatorsequenz aus Promotoren oder Enhancern bevorzugt eine genregulatorische Sequenz bzw. ein Element aus einem Gen verwendet, welches ein Protein kodiert, welches besonders stark oder selektiv in Zellen der Blutbildung exprimiert ist. Zu solchen genregulatorischen Sequenzen gehören Promotorsequenzen für Gene eines Cytokins oder seines Rezeptors, dessen Expression in den unreifen blutbildenden Zellen (oder in benachbarten Zellen, wie beispielsweise dem Stroma), dem nachfolgenden, auf die blutbildenden Zellen einwirkenden und als Wirksubstanz gewünschten Cytokin vorgeschaltet ist. Beispielsweise sind derartige auf unreife blutbildende Zellen einwirkende Cytokine:
- Stem Cell Factor
- IL-1
- IL-3
- IL-6
- GM-CSF

### 3.2. Auswahl der Strukturgene für Wirksubstanzen für blutbildende Zellen

Als Wirksubstanz im Sinne der Erfindung ist eine DNA-Sequenz zu verstehen, deren exprimiertes Protein die Proliferation und/oder Differenzierung von Blutzellen bewirkt.

### 4. Wirkstoff zur Therapie von Autoimmunerkrankungen, Allergien, Entzündungen und zur Verhütung von Organabstoßungen

### 4.1. Auswahl der Promotoren oder der Aktivatorsequenzen für Autoimmunerkrankungen u.a.

Als Promotoren oder Aktivatorsequenzen aus Promotoren oder Enhancern sind genregulatorische Sequenzen der Gene für solche Proteine zu verwenden, welche bei der Immunreaktion in Makrophagen und/oder in Lymphozyten verstärkt gebildet werden. Derartige Proteine sind beispielsweise:
- IL-1
- IL-1β
- IL-1-Rezeptor
- IL-2
- IL-2-Rezept̊or
- IL-3
- IL-3-Rezeptor
- IFNγ
- IL-4
- IL-4-Rezeptor
- IL-5
- IL-6
- LIF
- IL-7
- IL-10
- IL-11
- IL-12
- IL-13
- GM-CSF
- GM-CSF-Rezeptor
- Integrin beta 2 proteins

### 4.2. Auswahl der Gene für Wirksubstanzen für Autoimmunerkrankungen u.a.

Die Wirksubstanz im Sinne der Erfindung ist die DNA-Sequenz für ein Cytokin, ein Chemokin, einen Wachstumsfaktor oder einer ihrer Inhibitoren, für ein Ribozym katalytisch für das Transkriptionsprodukt eines dieser DNA-Sequenzen oder für das Transkriptionsprodukt eines Genes welches für ein Zellzykluskontrollprotein oder eine DNA-Sequenz für einen Antikörper oder ein Antikörperfragment oder für ein Enzym oder für ein Fusionsprotein eines Antikörpers, Cytokins oder Wachstumsfaktors mit einem Enzym kodiert. Die Auswahl der Wirksubstanz richtet sich nach der zu behandelnden Grunderkrankung und der gewählten Promotorsequenz.

### 5. Wirkstoff zur Behandlung der Arthritis

### 5.1. Auswahl der Promotoren oder der Aktivatorsequenzen für Arthritis

Im Sinne der Erfindung sind bevorzugt Promotoren, Aktivatorsequenzen aus Promotoren oder Enhancern oder genregulatorische Sequenzen solcher Gene zu verwenden, mit denen Transkriptionsfaktoren in aktiven Endothelzellen, Synovialzellen und Entzündungszellen interagieren. Im Sinne dieser Erfindung zählen zu den bevorzugten Promotorsequenzen genregulatorische Sequenzen bzw. Elemente aus Genen, die für besonders in Endothelzellen, Synovialzellen und Entzündungszellen exprimierte Proteine kodieren.

### 5.2. Auswahl der Strukturgene für Wirksubstanzen für Arthritis

Als Wirksubstanz im Sinne der Erfindung ist eine DNA-Sequenz zu verstehen, deren exprimiertes Protein die Entzündung beispielsweise im Gelenk direkt oder indirekt hemmt und/oder die Rekonstitution von extrazellulärer Matrix (Knorpel, Bindegewebe) im Gelenk fördert.

### 6. Herstellung eines Wirkstoffes gegen Infektionserreger

Der Wirkstoff kann in zwei grundsätzlich unterschiedlichen Formen hergestellt werden:
- für die Therapie von Virusinfektionen und Parasiteninvasionen oder aber
- für die Prophylaxe von Infektionserkrankungen durch Viren, Bakterien oder Parasiten.

Zur Prophylaxe von Infektionserkrankungen dienen Impfstoffe. Die Möglichkeiten, auf konventionellem Wege wirkungsvolle Impfstoffe herzustellen, sind jedoch beschränkt (Brown, Int. J. Technol. Assessm. Health Care 10, 161 (1994)), Ellis, Adv. Exp. Med. Biol. 327, 263 (1992)), Arnon et al., FASEB J. 6, 3265 (1992)).

Demzufolge wurde die Technologie der DNA-Vakzine entwickelt. Diese DNA-Vakzinen werten jedoch Fragen zur Wirksamkeitsstärke, Sicherheit und zu Nebenwirkungen auf (Fynan et al., Int. J. Immunopharm. 17, 79 (1995), Donnelly et al., Immunol. 2, 20 (1994)).

Wirkstoffe zur Prophylaxe von Infektionserkrankungen im Sinne dieser Erfindung zeichnen sich wegen ihrer Zellspezifität und Zellzyklusregulation durch ein hohes Maß an Sicherheit aus.

### 6.1. Auswahl der Promotor oder Aktivatorsequenzen

### 6.1.a) zur Therapie von Infektionserkrankungen

Als Aktivatorsequenz sind Promotorsequenzen von Zellgenen auszuwählen, deren Aktivität besonders durch Infektionen mit Bakterien oder Parasiten verändert werden oder es sind Promotorsequenzen solcher Viren auszuwählen, die die von ihnen infizierten Zellen transformieren und zur Proliferation anregen.

Zu diesen Viren gehören beispielsweise HBV, HCV, HSV, HPV, HIV, EBV und HTLV.

### 6.2. Auswahl der Strukturgene für Wirksubstanzen

### 6.2.a) zur Therapie von Infektionserkrankungen

Als Wirksubstanz ist die DNA eines Proteins auszuwählen, welches zytostatische, zytotoxische oder antivirale Wirkungen aufweist. Beispiele für zytotoxische oder zytostatische Proteine wurden schon oben aufgeführt. Bei der Wahl eines Enzyms ist nachfolgend die durch dieses Enzym spaltbare Vorstufe einer antiviralen zytotoxischen oder antiparasitären Substanz zu verabreichen.

Wirksubstanzen für antivirale Proteine im Sinne dieser Erfindung sind des weiteren antiviral wirksame Cytokine und Wachstumsfaktoren. Hierzu zählen beispielsweise die DNA-Sequenzen für folgende Wirksubstanzen:
- IFNα
- IFNβ
- IFNγ
- TNFβ
- TNFα
- IL-1
- TGFβ.

Als Wirksubstanz im Sinne der Erfindung können jedoch auch DNA-Sequenzen für Fusionsproteine zwischen den aufgeführten Cytokinen, Wachstumsfaktoren oder dem extrazellulären Teil der Rezeptoren zum einen und einem Liganden zum anderen Verwendung finden, zum Beispiel wurden Fusionsproteine mit dem Fc-Teil des menschlichen Immunglobulins in der EPA 0 464 633 A1 beschrieben.

Als Wirksubstanzen gelten des weiteren Gene für Ribozyme, welche die mRNA von Genen für Zellzykluskontrollproteine oder die mRNA von Viren verdauen. Ribozyme katalytisch für HIV wurden beispielsweise von Christoffersen et al., J. Med. Chem. 38, 2033 (1995) übersichtlich beschrieben.

Des weiteren ist eine Wirksubstanz im Sinne dieser Erfindung die DNA-Sequenz für einen Antikörper einer Spezifität, die das jeweilige Virus inaktiviert oder dessen VH und VL enthaltende Fragmente oder dessen über einen Linker verbundene VH und VL Fragmente, hergestellt beispielsweise entsprechend der von Marasco et al. (Proc. Natl. Acad. Sci. ♂USA 90, 7889 (1993)) beschriebenen Methodik. Beispiele für Antikörper einer derartigen Spezifität gegen Viren werden im Abschnitt 8.4. aufgeführt.

### 6.2.b) zur Prophylaxe von Infektionserkrankungen

Als Wirksubstanz ist die DNA eines vom Infektionserreger gebildeten Proteins auszuwählen, welches durch Auslösung einer Immunreaktion, d.h. durch Antikörperbindung und/oder durch zytotoxische T-Lymphozyten zur Neutralisierung und/oder zur Abtötung des Erregers führt. Derartige sogenannte Neutralisationsantigene werden als Impfantigene bereits angewandt (siehe Übersicht bei Ellis, Adv. Exp. Med. Biol. 327, 263 (1992)). Beispiele für DNA-Sequenzen, die Neutralisationsantigene kodieren, sind durch die folgenden Arbeiten zugänglich:
- Influenza A-Virus-Antigen
   (Ulmer et al., Science 259, 1745 (1993), Robinson et al., Vaccine 11, 957 (1993), Fynan et al., Int. J. Immunopharmac. 17, 79 (1995))
- HIV-Antigene
   (Wang et al., PNAS USA 90, 4156 (1993))
- Tollwut-Virus-Antigen
   (Donnelly et al., Immunol. 2/1, 20 (1994))
- HSV (Herpes Simplex Virus)-Antigen
   (Fleckenstein et al., Nature 274, 57 (1978))
- RSV (Respiratory Syncytial Virus)-Antigen
   (Du et al., Bio/Tech. 12, 813 (1994), Hall, Science 265, 1393 (1993))
- Parainfluenza-Virus-Antigen
   (Du et al., Bio/Techn. 12, 813 (1994))
- Rotavirus-Antigen
   (Albert et al., J. Clin. Microbiol. 25, 183 (1987), Anderson et al., J. Infect. Dis. 153, 823 (1986), Battaglia et al., J. Infect. Dis. 155, 140 (1987), Chanock et al., J. Infect. Dis. 148, 49 (1983), Dyall-Smith et al., J. Virol. 38, 1099 (1981), Glass et al., Science 265, 1389 (1994))
- VZV (Varizella Zoster Virus)-Antigen
   (Straus et al., Ann. Intern. Med. 109, 438 (1988), Gershon, Pediatr. Infect. Dis. 2, 171 (1991), Kinchington et al., J. Virol. 64, 4540 (1990))
- CMV (Cytomegalo-Virus)-Antigen
   (Plotkin, Science 265, 1383 (1994))
- Masern-Virus-Antigen
   (Katz und Kellin, Science 265, 1391 (1994))
- HPV (Humanes Papillomvirus)-Antigen
   (Tindl und Frazer, Curr. Topics Microbiol. Immunol. 186, 217 (1994))
- HBV (Hepatitis B-Virus)-Antigen
   (Valenzuela et al., Nature 280, 815 (1979), Heerman et al., J. Virol. 52, 396 (1984))
- HCV (Hepatitis C-Virus)-Antigen
   (Cerny et al., Curr. Topics Microbiol. Immunol. 189, 169 (1994), Esteban et al., Progr. Liver Dis. 10, 253 (1992), Jung et al., Eur. J. Clin. Invest. 24, 641 (1994))
- HDV (Hepatitis D-Virus)-Antigen
   (Iwarson, Scand. J. Infect. Dis. 24, 129 (1992), Consolo et al., Nephron. 61, 251 (1992))
- HEV (Hepatitis E-Virus)-Antigen
   (Iwarson, Scand. J. Infect. Dis. 24, 129 (1992), Consolo et al., Nephron. 61, 251 (1992))
- HAV (Hepatitis A-Virus)-Antigen
   (d'Hondt, Vaccine 10, 48 (1992), Andre, J. Infect. Dis. 171, 33 (1995), Lemon et al., Vaccine 10, 40 (1992), Melnick et al., Vaccine 10, 24 (1992), Flehmig, Baillieres Clin. Gastroenterol. 4, 707 (1990))
- Vibrio Cholera-Antigen
   (Levine und Kaper, Vaccine 11, 207 (1993))
- Borrelia Burgdorferi-Antigen
   (Schaible et al., Immunol. Letters 36, 219 (1993), Wallich et al., Lab. Med. 17, 669 (1993))
- Helicobacter pylori-Antigen
   (Crabtree et al., Lancet 338, 332 (1991), Blaser, J. Infect. Dis. 161, 626 (1990), Cover und Blaser, J. Biol. Chem. 267, 10570 (1993), Cover et al., Infect. Immunol. 58, 603 (1990), Dunn et al., J. Biol. Chem. 265, 9464 (1990), Dunn et al., Infect. Immunol. 60, 1946 (1992), Lage et al., Acta Gastroenterol. Belg. 56 (suppl.), 61 (1993), Mobley et al., Scand. J. Gastroint. 26 (suppl. 187), 39 (1991))
- Malaria-Antigen
   (Nussenzweig und Long, Science 265, 1381 (1994), Maurice, Science 267, 320 (1995), Enders et al., Vaccines 10, 920 (1992), Knapp et al., Infect. Imm. 60, 2397 (1992)).

Zu derartigen Wirksubstanzen im Sinne der Erfindung gehört jedoch auch die DNA eines Antiidiotyp-Antikörpers oder seiner Antigen-bindenden Fragmente, dessen Antigenbindungsstrukturen, die "complementarity determining regions", Kopien der Protein- oder Kohlenhydratstruktur des Neutralisationsantigens des Infektionserregers darstellen.

Derartige Antiidiotyp-Antikörper können besonders Kohlenhydratantigene bei bakteriellen Infektionserregern ersetzen.

Derartige antiidiotypische Antikörper und ihre Spaltprodukte wurden von Hawkins et al. (J. Immunother. 14, 273 (1993)) und Westerink und Apicella (Springer Seminars in Immunopathol. 15, 227 (1993)) übersichtlich beschrieben.

### 6.3. Kombination gleicher oder unterschiedlicher Wirksubstanzen für die Therapie oder Prophylaxe von Infektionserkrankungen

Gegenstand der Erfindung ist des weiteren ein Wirkstoff, in welchem eine Kombination der DNA-Sequenzen von gleichen Wirksubstanzen (A,A) oder unterschiedlichen Wirksubstanzen (A,B) vorliegt. Zur Expression von zwei Sequenzen ist vorzugsweise die cDNA einer "internal ribosome entry site" (IRES) als regulatorisches Element zwischengeschaltet.

Derartige IRES wurden beispielsweise von Montford und Smith (TIG 11, 179 (1995), Kaufman et al., Nucl. Acids Res. 19, 4485 (1991), Morgan et al., Nucl. Acids Res. 20, 1293 (1992), Dirks et al., Gene 128, 247 (1993), Pelletier und Sonnenberg, Nature 334, 320 (1988) und Sugitomo et al., BioTechn. 12, 694 (1994) beschrieben.

So kann die cDNA der IRES-Sequenz des Poliovirus (Position ♂ú 140 bis | 630 des 5' UTR (Pelletier und Sonenberg, Nature 334, 320 (1988)) zur Verknüpfung der DNA der antiviralen Substanz A (am 3'-Ende) und der DNA der antiviralen Substanz B (am 5'-Terminus) verwendet werden. Dies wird durch Figur 12 illustriert.

Ein derartiger Wirkstoff weist je nach Kombination additive (A+A, A+B1) oder synergistische Wirkung im Sinne der Erfindung auf.

So können beispielsweise für die Therapie von Viruserkrankungen zwei gleiche oder zwei unterschiedliche antivirale Wirksubstanzen miteinander kombiniert werden.

Bei der Prophylaxe von Infektionserkrankungen können mehrere Wirksubstanzen, die für unterschiedliche Antigene eines Infektionserregers oder unterschiedlicher Infektionserreger kodieren, miteinander kombiniert werden. Des weiteren kann die Wirksubstanz, die für das Antigen eines Infektionserregers kodiert, kombiniert werden mit einer Wirksubstanz, die kodiert für ein Cytokin oder einen Cytokinrezeptor.

Die sich so (nach Injektion des Wirkstoffes) gleichzeitig mit dem Infektionserregerantigen bildenden Cytokine oder Cytokinrezeptoren können Einfluß auf die Art und Stärke der sich entwickelnden Immunreaktion nehmen.

DNA-Sequenzen für Cytokine und Cytokinrezeptoren, welche die humorale Immunreaktion verstärken, sind bereits unter 6.2.d) beschrieben, solche zur Verstärkung der zellulären Immunreaktion unter 6.2.a) und 6.2.c).

DNA-Sequenzen für Cytokine, welche die Immunreaktion insgesamt verstärken, sind beispielsweise:
- ILl-1α
   (Fenton, Int. J. Immunopharm. 14, 401 (1992), Furntani et al., Nucl. Acids Res. 14, 3167 (1986), Lafage et al., Blood 73, 104 (1989), March et al., Nature 315, 641 (1985))
- IL-1β
   (Bensi et al., Gene 52, 95 (1987), Auron et al., PNAS 81, 7907 (1984), Clark et al., Nucl. Acids Res. 14, 7897 (1986))
- IL-2
   (Fletscher et al., Lymphok. Res. 6, 45 (1987), Matsui et al., Lymphokines 12, 1 (1985), Tanaguchi et al., Nature 302, 305 (1983))
- GM-CSF
   (Gough et al., Nature 309, 763 (1984), Nicola et al., J. Biol. Chem. 254, 5290 (1979), Wong et al., Science 228, 810 (1985))

### 7. Wirkstoff zur Behandlung von Leukämien und Tumoren

### 7.1. Auswahl der Promotoren oder Aktivatorsequenzen für Leukämien

Als Promotor oder Aktivatorsequenz ist eine genregulatorische Nukleotidsequenz vorgesehen, mit der Transkriptionsfaktoren, gebildet oder aktiv in Leukämiezellen oder Tumorzellen, interagieren.

Im Sinne dieser Erfindung zählen zu den bevorzugten Promotoren oder Aktivatorsequenzen genregulatorische Sequenzen bzw. Elemente aus Genen, die für besonders in Leukämiezellen oder Tumorsellen gebildete Proteine kodieren.

### 7.2. Auswahl der Strukturgene für Wirksubstanzen für Leukämien

Als Wirksubstanz im Sinne der Erfindung ist eine DNA-Sequenz zu verstehen, deren exprimiertes Protein direkt oder indirekt die Proliferation von Zellen, insbesondere auch von Leukämiezellen oder Tumorzellen, inhibiert. Zu diesen Wirksubstanzen gehören beispielsweise die DNA-Sequenzen für inhibitorische, zytostatische, apoptoseinduzierende oder zytotoxische Proteine oder von Enzymen, wie sie bereits beschrieben wurden.

Als Wirksubstanz ist des weiteren die DNA-Sequenz für ein Ribozym zu verstehen, welches die Aufspaltung der mRNA der Gene für Zellzykluskontrollproteine katalysiert.

### 8. Wirkstoff zur Inhibition der Proliferation glatter Muskelzellen bei Gefäßverschlüssen

### 8.1. Auswahl der Promotoren oder der Aktivatorsequenzen für glatte Muskelzellen

Als Promotoren oder Aktivatorsequenzen aus Promotoren oder Enhancern im Sinne der Erfindung sind bevorzugt genregulatorische Sequenzen bzw. Elemente aus Genen zu verwenden, die, besonders in glatten Muskelzellen, für gebildete Protein kodieren.

### 8.2. Auswahl der Strukturgene für Wirksubstanzen für glatte Muskelzellen

Als Wirksubstanz im Sinne der Erfindung ist eine DNA-Sequenz zu verstehen, deren exprimiertes Protein die Proliferation von glatten Muskelzellen inhibiert. Zu diesen Inhibitoren der Proliferation gehören die bereits unter 2.2.a) und 2.2.c) erwähnten Proteine.

Als Wirksubstanz ist jedoch auch die DNA-Sequenz für ein Enzym zu verstehen, welches eine inaktive Vorstufe eines Zytostatikums in ein Zytostatikum umwandelt (siehe 2.2.e)).

Als Wirksubstanz ist jedoch auch die DNA-Sequenz für ein Ribozym spezifisch für die mRNA von Genen für Zellzykluskontrollproteine zu verstehen (siehe 2.2.f)).

### 9. Wirkstoff zur Inhibition oder Förderung der Gerinnung

### 9.1. Auswahl der Promotoren oder der Aktivatorsequenzen zur Inhibition der Gerinnung

Als Promotoren oder Aktivatorsequenzen im Sinne der Erfindung sind bevorzugt genregulatorische Sequenzen bzw. Elemente aus Genen zu verwenden, welche in glatten Muskelzellen, in aktivierten Endothelzellen, in aktivierten Makrophagen oder in aktivierten Lymphozyten nachweisbare Proteine kodieren.

### 9.1.a) glatte Muskelzellen

Beispiele für Promotorsequenzen für Gene in glatten Muskelzellen sind bereits erwähnt.

### 9.1.b) aktivierte Endothelzellen

Beispiele für Proteine, welche besonders in aktivierten Endothelzellen gebildet werden, sind von Burrows et al. (Pharmac. Ther. 64, 155 (1994)) beschrieben worden. Im besonderen zählen zu diesen in Endothelzellen verstärkt auftretenden Proteinen beispielsweise solche Proteine, wie sie und die Promotorsequenzen ihrer Gene bereits oben aufgeführt wurden.

### 9.1.c) aktivierte Makrophagen und/oder aktivierte Lymphozyten

Als Aktivatorsequenz im Sinne dieser Erfindung sind außerdem Promotorsequenzen der Gene für Proteine zu verstehen, welche bei der Immunreaktion in Makrophagen und/oder in Lymphozyten verstärkt gebildet werden. Derartige Proteine sind bereits aufgeführt worden.

### 9.2. Auswahl der Strukturgene für Wirksubstanzen zur Inhibition oder Förderung der Gerinnung

Als Wirksubstanz zur Inhibition der Gerinnung im Sinne dieser Erfindung ist eine DNA-Sequenz zu verwenden, welche ein Protein kodiert, welches direkt oder indirekt die Thrombozytenaggregation oder einen Blutgerinnungsfaktor inhibiert oder die Fibrinolyse stimuliert.

Eine derartige Wirksubstanz wird als Gerinnungshemmer bezeichnet. Als Gerinnungshemmer sind Gene für beispielsweise Plasminogenaktivatoren (PA), so der Gewebe-PA (tPA) oder der Urokinase-ähnliche PA (uPA) oder Protein C, Antithrombin-III, C-1S-Inhibitor, µ1-Antitrypsin, der Tissue Factor Pathway Inhibitor (TFPI) oder Hirudin einzusetzen.

Als Wirksubstanz zur Förderung der Gerinnung im Sinne dieser Erfindung ist eine DNA-Sequenz zu verwenden, welche ein Protein kodiert, welches die Blutgerinnung direkt oder indirekt fördert. Zu diesen Proteinen gehören beispielsweise der Faktor VIII, der Faktor IX oder der Faktor XIII der Blutgerinnung.

### 10. Wirkstoff zum Schutz vor CNS-Schäden

### 10.1. Promotoren oder Aktivatorsequenzen aus Promotoren oder Enhancern für einen Wirkstoff zum Schutz vor CNS-Schäden

### 10.1.a) Promotoren oder Aktivatorsequenzen, aktiviert in Endothelzellen

Im besonderen zählen hierzu die Promotorsequenzen für die Gene von Endothelzell-spezifischen Proteinen.

### 10.1.b) Promotoren oder Aktivatorsequenzen, aktiviert in Gliazellen

Als bevorzugte Aktivatorsequenz ist des weiteren eine Nukleotidsequenz (Promotor- oder Enhancersequenz) zu verstehen, welche mit Transkriptionsfaktoren im besonderen Maße gebildet oder aktiv in Gliazellen interagiert.

### 10.2.) Wahl der Strukturgene für neurospezifische Faktoren

Als neurospezifische Faktoren im Sinne der Erfindung ist eine DNA-Sequenz zu verstehen, welche für einen neuronalen Wachstumsfaktor kodiert.

### Beispiele zur Erläuterung der Erfindung

Anhand der nachfolgenden Beispiele wird die vorliegende Erfindung näher erläutert.

### Beispiel 1

### Herstellung und Prüfung einer Aktivator-responsiven Promotoreinheit, wobei der Aktivator responsive Promotor die Bindesequenz für LexA darstellt.

Die erfindungsgemäße Aktivator-responsive Promotoreinheit besteht aus folgenden unterschiedlichen, stromabwärts aufeinanderfolgenden Nukleotidsequenzen:

### Aktivatorsubeinheit A

- der Promotor des cdc25C-Gens
   (Nukleinsäuren -290 bis +121; Zwicker et al., EMBO J. 14, 4514 (1995); Zwicker et al., Nucl. Acids Res. 23, 3822 (1995))
- der cDNA für die DNA-Bindedomäne des LexA-Proteins
   (Aminosäuren 1 bis 81; Kim et al., Science 255, 203 (1992)) oder das ganze LexA-Protein (Aminosäuren 1 bis 202; Brent et al., Cell 43, 729 (1985))
- die cDNA für Gal 80
   (Aminosäuren 1 bis 435; Leuther et al., Science 256, 1333 (1992))

### Aktivatorsubeinheit B

- der Promotor des von Willebrand Faktor-Gens
   (Nukleinsäuren -487 bis +247; Jahroudi and Lynch, Mol. Cell. Biol. 14, 999 (1994))
- der cDNA für die Gal80-Bindungsdomäne von Gal4
   (Aminosäuren 851 bis 881; Leuther et al., Science 256, 1333 (1992))
- dem nukleären Lokalisations-Signal (NLS) von SV40
   (SV40 Large T; Aminosäuren 126 bis 132: PKKKRKV; Dingwall et al., TIBS 16, 478 (1991))
- der sauren Transaktivierungsdomäne (TAD) von HSV-1 VP16
   (Aminosäuren 406 bis 488; Triezenberg et al., Genes Developm. 2, 718 (1988); Triezenberg, Curr. Op. Gen. Developm. 5, 190 (1995))

### Aktivator-responsiver Promotor

- der Bindesequenz für LexA (LexA-Operator) mit der Nukleotidsequenz
   SEQ ID NO.: 9
      5'-TACTGTATGTACATACAGTA-3'
   (Brent et al., Nature 612, 312 (1984)) gekoppelt an den basalen Promotor von SV40 (Nukleinsäuren 48 bis 5191; Tooze (ed.), DNA Tumor Viruses (Cold Spring Harbor New York, New York; Cold Spring Harbor Laboratory).

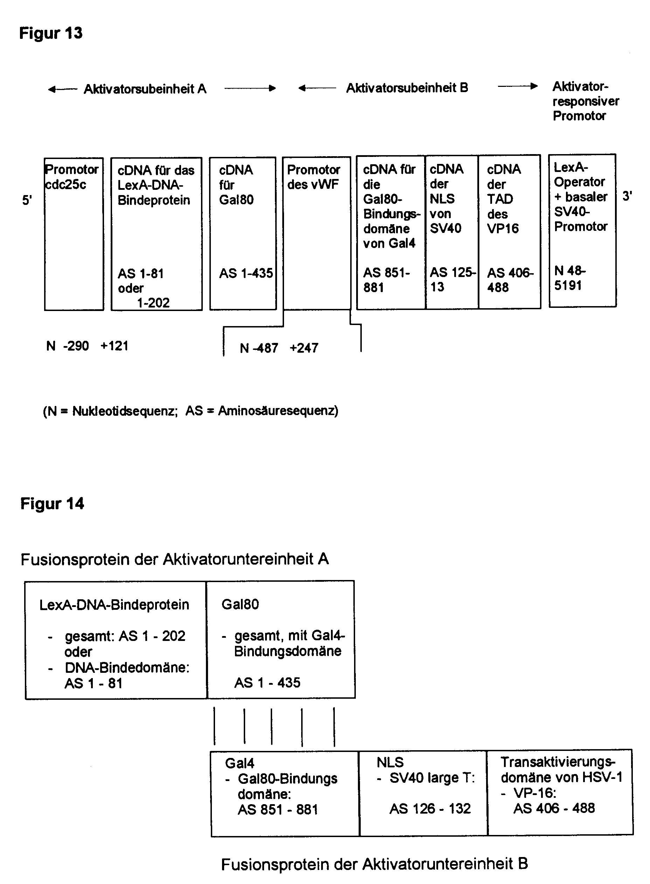

Die Reihenfolge der Nukleotidsequenzen der Aktivator-responsiven Promotoreinheiten ist in Figur 13 dargestellt.

Die Funktionsweise der beschriebenen Aktivatorsequenz ist wie folgt:
- Der Promotor cdc25C reguliert zellzyklusspezifisch die Transkription der kombinierten cDNA's für das Lex-DNA-Bindeprotein und für Gal80.
- Der Promotor der vWF beschränkt die Transkription der gekoppelten cDNA für die Gal80-Bindungsdomäne von Gal4, das NSL von SV40 und die TAP auf Endothelzellen.
- Die Expressionsprodukte der Aktivatorsubeinheiten A und B dimerisieren durch Bindung der Gal80-Bindungsdomäne von Gal4 an Gal80.

Die Dimerisierung ist schematisch in Figur 14 dargestellt.
- Das dimere Protein stellt einen chimären Transkriptionsfaktor für den Aktivator-responsiven Promotor "LexA-operator" dar.

Der Promotor ist nunmehr an seinem 3'-Ende mit der Sequenz GCCACC (Kocak, J. Cell. Biol. 108, 229 (1989)) und diese mit der cDNA für das Signalpeptid des Immunglobulins (Nukleotidsequenz ≤ 63 bis ≥ 107; Riechmann et al., Nature 332, 323 (1988)) verknüpft. An diese schließt sich die cDNA der β-Glucuronidase (Nukleotidsequenz ≤ 93 bis ≥ 1982; Oshima et al., PNAS USA 84, 685 (1987)) gemäß Figur 15 an.

Das so hergestellte Nukleotidkonstrukt wird in pUC18/19 oder Bluescript-abgeleiteten Plasmidvektoren einkloniert, die direkt oder in kolloidalen Dispersionssystemen für eine in vivo Applikation genutzt werden.

Die Verknüpfung der einzelnen Bestandteile des Konstruktes erfolgt über geeignete Restriktionsstellen, die über PCR-Amplifikation an den Termini der verschiedenen Elemente mitgeführt werden. Die Verknüpfung erfolgt mit Hilfe von dem Fachmann bekannten, für die Restriktionsstellen spezifischen Enzymen und DNA-Ligasen. Diese Enzyme sind käuflich zu erwerben.

Mit dem beschriebenen Plasmid werden in Kultur gehaltene menschliche Nabelschnurendothelzellen und Fibroblasten (Wi-38) mit dem Fachmann bekannter Methode (Lucibello et al., EMBO J. 14, 132 (1995)) transfiziert und die Menge an β-Glucuronidase, produziert von den Endothelzellen, mit Hilfe von 4-Methylumbelliferyl-β-glucuronid als Substrat gemessen.

Zur Überprüfung der Zellzyklusspezifität werden Endothelzellen durch Entzug von Methionin über 48 Stunden in G0/G1 synchronisiert (Nettelbeck et al., Publikation in Vorbereitung). Der DNA-Gehalt der Zellen wird nach Anfärbung mit Hoechst 33258 im Fluoreszenzaktivierungs-Cell Sorter bestimmt (Lucibello et al., EMBO J. 14, 132 (1995)).

Folgende Ergebnisse werden erzielt:

In transfizierten Fibroblasten kann keine Zunahme der β-Glucuronidase im Vergleich zu nichttransfizierten Fibroblasten ermittelt werden.

Transfizierte Endothelzellen exprimieren deutlich mehr β-Glucuronidase als nichttransfizierte Endothelzellen.

Proliferierende Endothelzellen (DNA > 2 S) sekretieren deutlich mehr β-Glucuronidase als in G0/G1 synchronisierte Endothelzellen (DNA = 2 S).

Somit führt die beschriebene Aktivator-responsive Promotoreinheit zu einer zellspezifischen, zellzyklusabhängigen Expression des Strukturgenes β-Glucuronidase.

Ein Wirkstoff gemäß der vorliegenden Erfindung ermöglicht nach lokaler Applikation beispielsweise an den Ort des Tumors oder nach intrakranieller bzw. subarachnoidaler Gabe oder systemischer, bevorzugt intravenöser oder intraarterieller Gabe, daß durch die Zellzyklus- und Endothelzellspezifität der Aktivator-responsiven Promotoreinheit vorwiegend, wenn nicht ausschließlich, nur proliferierende Endothelzellen β-Glucuronidase ausscheiden. Diese β-Glucuronidase spaltet ein nunmehr infiziertes gut verträgliches Doxorubicin-β-glucuronid (Jacquesy et al., EPO 0511 917 A1) in das zytostatisch wirkende Doxorubicin. Dieses hemmt die Endothelzellproliferation und wirkt zytostatisch auf diese Zellen wie auch auf benachbarte Tumorzellen. Hierdurch kommt es zur Hemmung des Tumorwachstums.

Da der Wirkstoff sowohl durch seine Zell- als auch Zellzyklusspezifität ein hohes Maß an Sicherheit verspricht, kann er auch in hohen Dosierungen und, falls notwendig, mehrmals in Abständen von Tagen oder Wochen zur Therapie von Tumorerkrankungen angewandt werden.

### Beispiel 2

### Herstellung eines multiplen Promotors mit einem nukleären Retentions-Signal (NRS) und einem nukleären Export-Faktor (NEF)

Der erfindungsgemäße multiple Promotor besteht aus folgenden unterschiedlichen, stromabwärts aufeinanderfolgenden Nukleotidsequenzen:

### Element A

- dem Promotor des cdc25C-Gens
   (Nukleinsäuren -290 bis +121; Zwicker et al., EMBO J. 14, 4514 (1995); Zwicker et al., Nucl. Acids. Res. 23, 3822 (1995))
- der Sequenz GCCACC
   (Kodak, J. Cell. Biol. 108, 229 (1989))
- der cDNA für das Signalpeptid des Immunglobulins
   (Nukleotidsequenz ≤ 63 bis ≥ 107; Riechmann et al., Nature 332, 323 (1988))
- der cDNA der β-Glucuronidase
   (Nukleotidsequenz ≤ 93 bis ≥ 1982; Oshima et al., PNAS USA 84, 685 (1987))
- der cDNA für RER von HIV-1 Virus als nukleäres Retentions-Signal (NRS)
   (Nukleotidsequenz 7357 bis 7602; Ratner et al., Nature 313, 277 (1985); Malim et al., Nature 338, 254 (1989)).

### Element B

- der Promotor des von Willebrand Faktor (vWF)-Gens
   (Nukleinsäuren -487 bis +247; Jahroudi and Lynch, Mol. Cell Biol. 14, 999, 1994))
- der cDNA für REV von HIV-1 Virus als nukleärer Export-Faktor (NEF)
   (Aminosäuresequenz 1-117; Ratner et al., Nature 313, 277 (1985)).

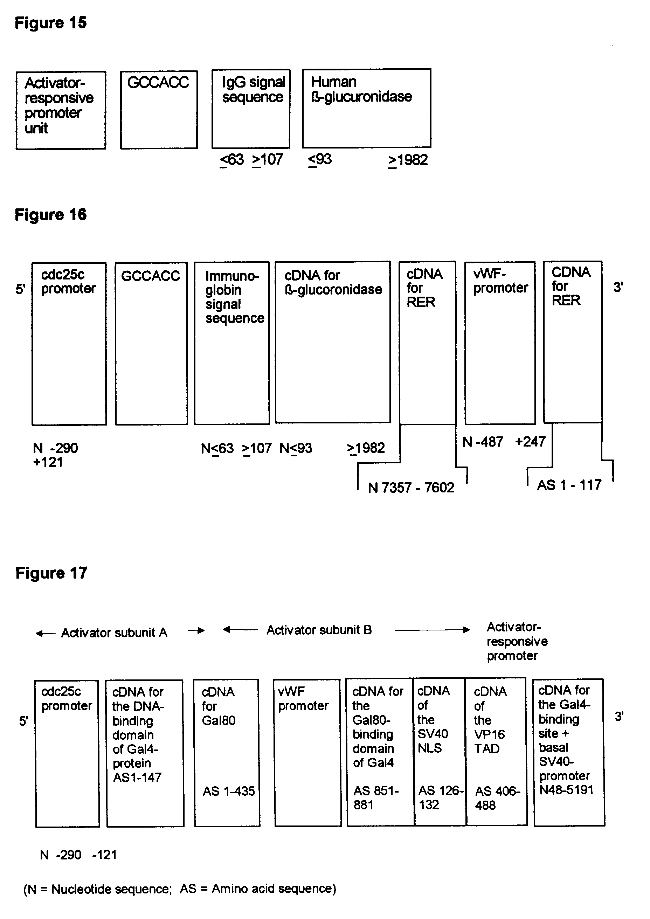

Die Nukleotidsequenzen von Element A und Element B sind nach Schema der Figur 16 verknüpft.

Das so hergestellte Nukleotidkonstrukt wird in pUC18/19 oder Bluescript-abgeleiteten Plasmidvektoren einkloniert, die direkt oder in kolloidalen Dispersionssystemen für eine in vivo Applikation genutzt werden.

Die Verknüpfung der einzelnen Bestandteile des Konstruktes erfolgt über geeignete Restriktionsstellen, die über PCR-Amplifikation an den Termini der verschiedenen Elemente mitgeführt werden. Die Verknüpfung erfolgt mit Hilfe von dem Fachmann bekannten, für die Restriktionsstellen spezifischen Enzymen und DNA-Ligasen. Diese Enzyme sind käuflich zu erwerben.

Mit dem beschriebenen Plasmid werden in Kultur gehaltene menschliche Nabelschnurendothelzellen und Fibroblasten (Wi-38) mit dem Fachmann bekannter Methode (Lucibello et al., EMBO J. 14, 132 (1995)) transfiziert und die Menge an β-Glucuronidase, produziert von den Endothelzellen, mit Hilfe von 4-Methylumbelliferyl-β-glucuronid als Substrat gemessen.

Zur Überprüfung der Zellzyklusspezifität werden Endothelzellen durch Entzug von Methionin über 48 Stunden in G0/G1 synchronisiert (Nettelbeck et al., Publikation in Vorbereitung). Der DNA-Gehalt der Zellen wird nach Anfärbung mit Hoechst 33258 im Fluoreszenzaktivierungs-Cell Sorter bestimmt (Lucibello et al., EMBO J. 14, 132 (1995)).

Folgende Ergebnisse werden erzielt:

In transfizierten Fibroblasten kann keine Zunahme der β-Glucuronidase im Vergleich zu nichttransfizierten Fibroblasten ermittelt werden.

Transfizierte Endothelzellen exprimieren deutlich mehr β-Glucuronidase als nichttransfizierte Endothelzellen.

Proliferierende Endothelzellen (DNA > 2 S) sekretieren deutlich mehr β-Glucuronidase als in G0/G1 synchronisierte Endothelzellen (DNA = 2 S).

Somit führt die beschriebene multiple Promotoreinheit zu einer zellspezifischen, zellzyklusabhängigen Expression des Strukturgenes β-Glucuronidase.

### Beispiel 3

### Herstellung einer Aktivator-responsiven Promotoreinheit, bei welcher der Aktivator-responsive Promotor die Bindesequenz für Gal4 darstellt.

Die erfindungsgemäße Aktivator-responsive Promotoreinheit besteht aus folgenden unterschiedlichen, stromabwärts aufeinanderfolgenden Nukleotidsequenzen:

### Aktivatorsubeinheit A

- der Promotor des cdc25C-Gens
   (Nukleinsäuren -290 bis +121; Zwicker et al., EMBO J. 14, 4514 (1995); Zwicker et al., Nucl. Acids Res. 23, 3822 (1995))
- der cDNA für die DNA-Bindedomäne des Gal4-Proteins
   (Aminosäuren 1 bis 147; Chasman und Kornberg, Mol. Cell Biol. 10, 2916 (1990))
- die cDNA für Gal80
   (Aminosäuren 1 bis 435; Leuther et al., Science 256, 1333 (1992))

### Aktivatorsubeinheit B

- der Promotor des von Willebrand Faktor Gens
   (Nukleinsäuren -487 bis +247; Jahroudi and Lynck, Mol. Cell. Biol. 14, 999 (1994))
- der cDNA für die Gal80-Bindungsdomäne von Gal4
   (Aminosäuren 851 bis 881; Leuther et al., Science 256, 1333 (1992))
- dem nuklearen Lokalisations-Signal (NLS) von SV40
   (SV40 Large T; Aminosäuren 126 bis 132: PKKKRKV; Dingwall et al., TIBS 16, 478 (1991))
- der sauren Transaktivierungsdomäne (TAD) von HSV-1 VP16
   (Aminosäuren 406 bis 488; Triezenberg et al., Genes Developm. 2, 718 (1988); Triezenberg, Curr. Opin. Gen. Developm. 5, 190 (1995))

### Aktivator-responsiver Promotor

- die Bindesequenz für Gal4 mit der Nukleotidsequenz
   5'-CGGACAACTGTTGAC CG-3' (Chasman und Kornberg, Mol. Cell Biol. 10, 2916 (1999)) gekoppelt an den basalen Promotor von SV40 (Nukleinsäuren 48 bis 5191; Tooze (ed.), DNA Tumor Viruses (Cold Spring Harbor New York, New York; Cold Spring Harbor Laboratory).

Die Reihenfolge der Nukleotidsequenzen der Aktivator-responsiven Promotoreinheiten ist in Figur 17 dargestellt.

Die Funktionsweise der beschriebenen Aktivatorsequenz ist wie folgt:
- Der Promotor cdc25C reguliert zellzyklusspezifisch die Transkription der kombinierten cDNA's für das Gal4-Bindeprotein und für Gal80.
- Der Promotor des vWF-Gens beschränkt die Transkription der gekoppelten cDNA für die Gal80-Bindungsdomäne von Gal4, das NSL von SV40 und die TAP auf Endothelzellen.
- Die Expressionsprodukte der Aktivatorsubeinheiten A und B dimerisieren durch Bindung der Gal80-Bindungsdomäne von Gal4 an Gal80.

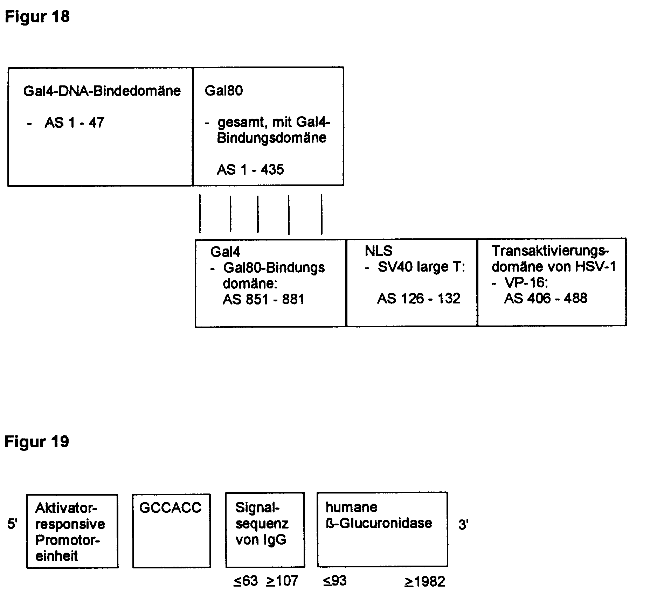

Die Dimerisierung ist schematisch in Figur 18 dargestellt.
- Das dimere Protein stellt einen chimären Transkriptionsfaktor für den Aktivator-responsiven Promotor (DNA-Sequenz für die Gal4-Bindedomäne/SV40-Promotor) dar.

Der Promotor ist nunmehr an seinem 3'-Ende mit der Sequenz GCCACC (Kocak, J. Cell Biol. 108, 229 (1989)) und diese mit der cDNA für das Signalpeptid des Immunglobulins (Nukleotidsequenz ≤ 63 bis ≥ 107; Riechmann et al., Nature 332, 323 (1988)) verknüpft. An diese schließt sich die cDNA der β-Glucuronidase (Nukleotidsequenz ≤ 93 bis ≥ 1982; Oshima et al., PNAS USA 84, 685 (1987)) gemäß von Figur 19 an.

Das so hergestellte Nukleotidkonstrukt wird in pUC18/19 oder Bluescript-abgeleiteten Plasmidvektoren einkloniert, die direkt oder in kolloidalen Dispersionssystemen für eine in vivo Applikation genutzt werden.

Die Verknüpfung der einzelnen Bestandteile des Konstruktes erfolgt über geeignete Restriktionsstellen, die über PCR-Amplifikation an den Termini der verschiedenen Elemente mitgeführt werden. Die Verknüpfung erfolgt mit Hilfe von dem Fachmann bekannten, für die Restriktionsstellen spezifischen Enzymen und DNA-Ligasen. Diese Enzyme sind käuflich zu erwerben.

Mit dem beschriebenen Plasmid werden in Kultur gehaltene menschliche Nabelschnurendothelzellen und Fibroblasten (Wi-38) mit dem Fachmann bekannter Methode (Lucibello et al., EMBO J. 14, 132 (1995)) transfiziert und die Menge an β-Glucuronidase, produziert von den Endothelzellen, mit Hilfe von 4-Methylumbelliferyl-β-glucuronid als Substrat gemessen.

Zur Überprüfung der Zellzyklusspezifität werden Endothelzellen durch Entzug von Methionin über 48 Stunden in G0/G1 synchronisiert (Nettelbeck et al., Publikation in Vorbereitung). Der DNA-Gehalt der Zellen wird nach Anfärbung mit Hoechst 33258 im Fluoreszenzaktivierungs-Cell Sorter bestimmt (Lucibello et al., EMBO J. 14, 132 (1995)).

Folgende Ergebnisse werden erzielt:

In transfizierten Fibroblasten kann keine Zunahme der β-Glucuronidase im Vergleich zu nichttransfizierten Fibroblasten ermittelt werden.

Transfizierte Endothelzellen exprimieren deutlich mehr β-Glucuronidase als nichttransfizierte Endothelzellen.

Proliferierende Endothelzellen (DNA >2 S) sekretieren deutlich mehr β-Glucuronidase als in G0/G1 synchronisierte Endothelzellen (DNA = 2 S).

Somit führt die beschriebene Aktivator-responsive Promotoreinheit zu einer zellspezifischen, zellyzklusabhängigen Expression des Strukturgens β-Glucuronidase.

### Beispiel 4

### Herstellung und Prüfung einer weiteren Aktivator-responsiven Promotoreinheit, bei welcher der Aktivator-responsive Promotor die Bindesequenz für Gal4 darstellt.

Diese erfindungsgemäße Aktivator-responsive Promotoreinheit besteht aus folgenden unterschiedlichen, stromabwärts aufeinanderfolgenden Nukleotidsequenzen:

### Aktivatorsubeinheit A

- der Promotor des cdc25C Genes (Nukleinsäuren -290 bis +121; Zwicker et al., EMBO J. 14, 4514 (1995); Zwicker et al., Nucl. Acids Res. 23, 3822 (1995))
- dem nuklearen Lokalisationssignal (NLS) von SV40 (SV40 Large T, Aminosäuren 126-132; PKKKRKV, Dingwall et al., TIBS 16, 478 (1991))
- der sauren Transaktivierungsdomäne (TAD) von HSV-1 VP16 (Aminosäuren 406 bis 488; Triezenberg et al., Genes Developm. 2, 718 (1988); Triezenberg, Curr. Opin. Gen. Developm. 5, 190 (1995))
- der cDNA für den zytoplasmatischen Teil des CD4 Glycoproteins (Aminosäuren 397-435; Simpson et al., Oncogene 4, 1141 (1989); Maddon et al., Cell 42, 93 (1985))

### Aktivatorsubeinheit B

- dem Promotor des cdc25C Gens (Nukleinsäuren -290 bis +121; Zwicker et al., EMBO J. 14, 4514 (1995); Zwicker et al., Nucl. Acids Res. 23, 3822 (1995))
- dem nuklearen Lokalisationssignal (NLS) von SV40 (SV40 Large T; Aminosäuren 126-132 PKKKRKV; Dingwall et al., TIBS 16, 478 (1991))
- der cDNA für die DNA-Bindedomäne des Gal4 Proteins (Aminosäuren 1 bis 147, Chasman und Kornberg, Mol. Cell. Biol. 10, 2916 (1990))
- der cDNA für die CD4 Bindesequenz des p56 lck Proteins (Aminosäuren 1-71; Shaw et al., Cell 59, 627 (1989); Turner et al., Cell 60, 755 (1990); Perlmutter et al., J. Cell. Biochem. 38, 117 (1988))

### Aktivator-responsive Promotoren

- 10x die Bindesequenz für Gal4-Bindeprotein mit der Nukleotidsequenz 5'-CGGACAATGTTGACCG-3' (Chasman und Kornberg, Mol. Cell. Biol. 10, 2916 (1989))
- der basale Promotor von SV40 (Nukleinsäuren 48 bis 5191; Tooze (ed). DNA Tumor Viruses (Cold Spring Harbor New York, New York, Cold Spring Harbor Laboratory)

### Effektorgen

- die cDNA für Luciferase (Reportergen) (Nordeen BioTechniques 6, 454 (1988))

Die Funktionsweise der beschriebenen Aktivatorsequenz ist wie folgt:
- Der Promotor cdc25C reguliert zellzyklusspezifisch die Transkription der kombinierten cDNAs für die Aktivierungsdomäne von VP16 und dem zytoplasmatischen Teil von CD4 (Aktivierungsuntereinheit A).
- Der Promotor cdc25C reguliert des weiteren die Transkription der kombinierten cDNAs für das DNA Bindeprotein von Gal4 und des CD4 bindenden Teiles des p56 lck-Proteins (Aktivierungsuntereinheit B).
- Die Expressionsprodukte der Aktivatorsubeinheiten A und B dimerisieren durch Bindung der CD4-Domäne an die p56 lck-Domäne.
- Das dimere Protein stellt einen chimären Transkriptionsfaktor für den Aktivator-responsiven Promotor (DNA-Sequenz für die Gal4-Bindedomäne/SV40 Promotor) für die Transkription des Effektorgenes (= Reportergen = Luciferasegen) dar.

Das so hergestellte Nukleotidkonstrukt wird in den pXP2 Plasmidvektor (Nordeen, BioTechniques 6, 454 (1988)) einkloniert, die direkt oder in kolloidalen Dispersionssystemen für eine in vivo Applikation genutzt werden.

Die Verknüpfung der einzelnen Bestandteile des Konstruktes erfolgt über geeignete Restriktionsstellen, die über PCR-Amplifikation an den Termini der verschiedenen Elemente mitgeführt werden. Die Verknüpfung erfolgt mit Hilfe von dem Fachmann bekannten, für die Restriktionsstellen spezifischen Enzyme und DNA-Ligasen. Diese Enzyme sind käuflich zu erwerben.

Mit dem beschriebenen Plasmid werden in Kultur gehaltene 3T3 Fibroblasten mit dem Fachmann bekannter Methode (Lucibello et al., EMBO J. 14, 132 (1995)) transfiziert und die Menge an Luciferase produziert von den Fibroblasten, wie von Herber et al. (Oncogene 9, 1295 (1994)) und Lucibello et al. (EMBO J. 14, 132 (1995)) beschrieben, gemessen.

Zur Überprüfung der Zellzyklusspezifität werden die Fibroblasten durch Entzug von Serum über 48 Stunden in G0/G1 synchronisiert. Der DNA-Gehalt der Zellen wird nach Anfärbung mit Hoechst 33258 im Fluoreszenzaktivierungs-Cell Sorter bestimmt (Lucibello et al., EMBO J. 14, 132 (1995)).

Folgende Ergebnisse werden erzielt:

In transfizierten Fibroblasten kann eine deutliche Zunahme der Luciferase im Vergleich zu nichttransfizierten Fibroblasten ermittelt werden.

Proliferierende Fibroblasten (DNA > 2S) bilden deutlich mehr Luciferase als in G0/G1 synchronisierte Fibroblasten (DNA = 2 S).

Somit führt die beschriebene Aktivator-responsive Promotoreinheit zu einer zellzyklusabhängigen Expression des Reportergenes Luciferase.

Ein Wirkstoff gemäß der vorliegenden Erfindung ermöglicht nach lokaler Applikation beispielsweise an den Ort des Tumors oder nach intrakranieller bzw. subarachnoidaler Gabe oder systemischer, bevorzugt intravenöser oder intraarterieller Gabe, daß durch die Zellzyklus- und Endothelzellspezifität der Aktivator-responsiven Promotoreinheit vorwiegend, wenn nicht ausschließlich, nur proliferierende Endothelzellen β-Glucuronidase ausscheiden. Diese β-Glucuronidase spaltet ein nunmehr infiziertes gut verträgliches Doxorubicin-β-glucuronid (Jacquesy et al., EPO 0511 917 A1) in das zytostatisch wirkende Doxorubicin. Dieses hemmt die Endothelzellproliferation und wirkt zytostatisch auf diese Zellen wie auch auf benachbarte Tumorzellen. Hierdurch kommt es zur Hemmung des Tumorwachstums.

### Figurenlegende:

- Figur 1:: Selbsterklärendes Schema eines erfindungsgemäßen Nukleinsäurekonstrukts.
- Figur 2:: Selbsterklärendes Schema eines erfindungsgemäßen Nukleinsäurekonstrukts.
- Figur 3:: Selbsterklärendes Schema eines erfindungsgemäßen Nukleinsäurekonstrukts.
- Figur 4:: Selbsterklärendes Schema eines erfindungsgemäßen Nukleinsäurekonstrukts.
- Figur 5:: Selbsterklärendes Schema eines erfindungsgemäßen Nukleinsäurekonstrukts.
- Figur 6:: Selbsterklärendes Schema eines erfindungsgemäßen Nukleinsäurekonstrukts.
- Figur 7:: Selbsterklärendes Schema eines erfindungsgemäßen Nukleinsäurekonstrukts.
- Figur 8:: Selbsterklärendes Schema eines erfindungsgemäßen Nukleinsäurekonstrukts.
- Figur 9:: Selbsterklärendes Schema eines erfindungsgemäßen Nukleinsäinrekonstrukts.
- Figur 10:: Selbsterklärendes Schema eines erfindungsgemäßen Nukleinsäurekonstrukts.
- Figur 11:: Selbsterklärendes Schema eines erfindungsgemäßen Nukleinsäurekonstrukts.
- Figur 12:: Selbsterklärendes Schema eines erfindungsgemäßen Nukleinsäurekonstrukts.
- Figur 13:: Selbsterklärendes Schema eines erfindungsgemäßen Nukleinsäurekonstrukts.
- Figur 14:: Selbsterklärendes Schema eines erfindungsgemäßen Nukleinsäurekonstrukts.
- Figur 15:: Selbsterklärendes Schema eines erfindungsgemäßen Nukleinsäurekonstrukts.
- Figur 16:: Selbsterklärendes Schema eines erfindungsgemäßen Nukleinsäurekonstrukts.
- Figur 17:: Selbsterklärendes Schema eines erfindungsgemäßen Nukleinsäurekonstrukts.
- Figur 18:: Selbsterklärendes Schema eines erfindungsgemäßen Nukleinsäurekonstrukts.
- Figur 19:: Selbsterklärendes Schema eines erfindungsgemäßen Nukleinsäurekonstrukts.

## Patentansprüche

1. Nukleinsäurekonstrukt, dadurch gekennzeichnet, daß es ein nukleäres Retentions-Signal aufweist, das in Leserichtung stromabwärts mit einem Transgen verknüpft ist.

2. Nukleinsäurekonstrukt nach Anspruch 1 dadurch gekennzeichnet, daß es folgende Komponenten in Leserichtung vom 5'-Ende zum 3'-Ende aufweist:
a) eine erste unspezifische, zellspezifische, virusspezifische, metabolisch und/oder zellzyklusspezifisch aktivierbare Promotor- oder Enhancersequenz, die die basale Transkription eines Transgenes aktiviert,
b) ein Transgen, das als ein Strukturgen für einem Wirkstoff kodiert,
c) ein nukleäres Retentions-Signal, dessen cDNA am 5'-Ende mit dem 3'-Ende des Strukturgenes mittelbar oder unmittelbar verknüpft ist.

3. Nukleinsäurekonstrukt nach Anspruch 1 oder 2, dadurch gekennzeichnet, daß das Transkriptionsprodukt des nukleären Retentions-Signals eine Bindestruktur für einen nukleären Export-Faktor aufweist.

4. Nukleinsäurekonstrukt nach einem der Ansprüche 2 oder 3, dadurch gekennzeichnet, daß es zusätzlich zu den Komponenten a) bis c) folgende Komponenten aufweist:
d) eine weitere Promotor- oder Enhancersequenz, welche die basale Transkription eines nukleären Export-Faktores aktiviert und
e) eine Nukleinsäure kodierend für einen nukleären Export-Faktor, der an das Transkriptionsprodukt des nukleären Retentions-Signals (c) bindet und hierdurch den Transport des Transkriptionsproduktes des Transgens aus dem Zellkern in das Zytoplasma vermittelt.

5. Nukleinsäurekonstrukt nach Anspruch 4, dadurch gekennzeichnet, daß die erste Promotor- oder Enhancersequenz (a) und die zweite Promotor- oder Enhancersequenz (d) gleich oder unterschiedlich sind und, daß mindestens eine der Komponenten a) und d) unspezifisch, zellspezifisch, virusspezifisch, metabolisch, insbesondere durch Hypoxie, oder zellzyklusspezifisch aktivierbar ist.

6. Nukleinsäurekonstrukt nach Anspruch 4 oder 5, dadurch gekennzeichnet, daß wenigstens eine der Promotor- oder Enhancersequenzen (a) und (d) ein chimärer Promotor ist, bei welchem das Promotormodul CDE-CHR oder E2FBS-CHR mit einer stromaufwärts benachbarten zellspezifisch, virusspezifisch oder metabolisch aktivierbaren Aktivatorsequenz interagieren und dadurch die Expression eines stomabwärts gelegenen Gens beeinflussen, insbesondere inhibieren kann.

7. Nukleinsäurekonstrukt nach Anspruch 3 oder 4, dadurch gekennzeichnet, daß wenigstens eine der Komponenten a) und d) eine Aktivator-responsive Promotoreinheit darstellt umfassend folgende Komponenten:
f) wenigstens eine Promotor- oder Enhancersequenz, die unspezifisch, virusspezifisch, metabolisch, zellspezifisch und/oder zellzyklusspezifisch aktivierbar ist und
g) wenigstens eine Aktivatorsubeinheit, die stromabwärts von der Promotor- oder Enhancersequenz (f) gelegen ist und durch die Promotor- oder Enhancersequenz (f) in ihrer basalen Transkription aktiviert wird,
h) ein Aktivator-responsiver Promotor, welcher durch die Expressionsprodukte von einer Aktivatorsubeinheit (g) oder mehreren gleichen oder unterschiedlichen Aktivatorsubeinheiten (g, g') aktiviert wird.

8. Nukleinsäurekonstrukt nach Anspruch 7, bei welchem die Promotor- oder Enhancersequenz (a) und/oder (d) und/oder der Aktivator-responsive Promotor ein chimärer Promotor ist und die Aktivatorsubeinheit (g) ein Gen für wenigstens einen Transkriptionsfaktor darstellt, der den chimären Promotor des Aktivator-responsiven Promotors (h) aktiviert.

9. Nukleinsäurekonstrukt nach Anspruch 7, dadurch gekennzeichnet, daß
- der Aktivator-responsive Promotor (h) der LexA-Operator in Verbindung mit dem SV40 Promotor ist und
- die Aktivatorsubeinheit (g) die cDNA für das LexA-DNA-Bindeprotein kodierend für die Aminosäuren 1-81 oder 1-202 umfaßt, deren 3'-Ende verknüpft ist mit dem 5'-Ende der cDNA für das Gal80-Protein (Aminosäuren 1-435) darstellt und
- eine weitere Aktivatorsubeinheit (g') die cDNA der Gal80-Bindungsdomäne des Gal4-Proteins kodierend für die Aminosäuren 851-881 enthält, deren 3'-Ende verknüpft ist mit dem 5'-Ende der cDNA des SV40 large T-Antigens kodierend für die Aminosäuren 126-132, deren 3'-Ende verknüpft ist mit dem 5'-Ende der cDNA für die Transaktivierungsdomäne des VP16 von HSV-1 kodierend für die Aminosäuren 406-488.
- oder daß der Aktivator-responsive Promotor (h) eine oder mehrere Bindesequenzen für das Gal4-Protein in Verbindung mit mindestens einem SV40-Promotor enthält und die Aktivatorsubeinheiten (g, g') entweder enthalten:
• Aktivatorsubeinheit g: die cDNA für die DNA-Bindedomäne des Gal4-Proteins (Aminosäuren 1 bis 147) und die cDNA für Gal80 und die
• Aktivatorsubeinheit g': die cDNA für die Gal80-Bindedomäne von Gal4 (Aminosäuren 851 bis 881); die cDNA für das nukleare Lokalisationssignal (NLS) von SV40 (Aminosäuren 126 bis 132) und die cDNA für die saure Transaktivierungsdomäne von HSV-1 VP16 (Aminosäuren 406 bis 488)
- oder aber enthalten
• Aktivierungssubeinheit g: die cDNA für das nukleare Lokalisationssignal von SV40 (Aminosäuren 126-132); die cDNA für die saure Transaktivierungsdomäne von HSV-1 VP16 (Aminosäuren 406 bis 488) und die cDNA für den zytoplasmatischen Teil des CD4 Glykoproteins (Aminosäuren 397-435) und die
• Aktivierungssubeinheit g: die cDNA für das nukleare Lokalisationssignal von SV40 (Aminosäuren 126-132), die cDNA für die DNA-Bindedomäne des Gal4-Proteins (Aminosäuren 1 bis 147 und die cDNA für die CD4-Bindesequenz des p56 lck-Proteins (Aminosäuren 1-71).

10. Nukleinsäurekonstrukt nach einem der vorhergehenden Ansprüche, dadurch gekennzeichnet, daß das Gen kodierend für das nukleäre Retentions-Signal (c) ausgewählt ist aus der Gruppe umfassend das Rev-responsive Element (RRE) von HIV-1 oder HIV-2, das RRE-äquivalente Retentions-Signal von Retroviren oder das RRE-äquivalente Retentions-Signal des HBV.

11. Nukleinsäurekonstrukt nach Anspruch 4, dadurch gekennzeichnet, daß der nukleäre Export-Faktor (e) ein Gen ist ausgewählt aus der Gruppe umfassend das Rev-Gen der Viren HIV-1, HIV-2, Visna-Maidi Virus, Caprine arthritis encephalitis Virus, das Virus der infektiösen Anämie des Pferdes, das Immundefizienzvirus der Katze, von Retroviren, von HTLV oder das Gen des hnRNP-A1-Proteins oder das Gen des Transkriptionsfaktors TFIII-A.

12. Nukleinsäurekonstrukt nach einem der vorhergehenden Ansprüche, dadurch gekennzeichnet, daß es sich bei der Nukleinsäure um DNS handelt.

13. Nukleinsäurekonstrukt nach einem der vorhergehenden Ansprüche, dadurch gekennzeichnet, daß es sich bei dem Nukleinsäurekonstrukt um einen Vektor handelt.

14. Nukleinsäurekonstrukt nach Anspruch 13, dadurch gekennzeichnet, daß es sich um einen Plasmidvektor handelt.

15. Nukleinsäurekonstrukt nach Anspruch 13, dadurch gekennzeichnet, daß es sich um einem viralen Vektor handelt.

16. Nukleinsäurekonstrukt nach einem der vorhergehenden Ansprüche, dadurch gekennzeichnet, daß es sich bei dem Transgen (b) um ein Strukturgen handelt, das für einen Wirkstoff kodiert, der ausgewählt ist aus der Gruppe umfassend Cytokine, Wachstumsfaktoren, Antikörper oder Antikörperfragmente, Fusionsproteine aus einem Liganden und einem Enzym, einem Cytokin oder Wachstumsfaktor, Rezeptoren für Cytokine oder Wachstumsfaktoren, antiproliferativ, apoptotisch oder zytotoxisch wirkende Proteine, Angiogeneseinhibitoren und/oder Thrombose-induzierenden Proteinen, Blutgerinnungsfaktor, Gerinnungshemmer, Fibrinolyse induzierendes Protein Komplement aktivierende Proteine wie Cobra Venum Faktor; humanes C3b, modifiziertes C3b; bakterielle Proteine, Virushüllproteine, bakterielle Antigene und parasitäre Antigene, Blut-Kreislauf wirkende Peptide oder Proteine und Ribozyme.

17. Nukleinsäurekonstrukt nach Anspruch 16, dadurch gekennzeichnet, daß es sich bei dem Transgen (b) um ein Strukturgen handelt, das für ein Ribozym kodiert, welches die mRNA inaktiviert, welches kodiert für ein Protein ausgewählt aus der Gruppe umfassend Zellzykluskontrollproteine, insbesondere Cyclin A, Cyclin B, Cyclin D1, Cyclin E, E2F1-5, cdc2, cdc25C oder DP1, Virusproteine oder Cytokine oder Wachstumsfaktoren oder deren Rezeptoren.

18. Nukleinsäurekonstrukt nach einem der vorhergehenden Ansprüche, dadurch gekennzeichnet, daß die Promotor-, Enhancer- oder Aktivatorsequenz ausgewählt ist aus der Gruppe von genregulatorischen Nukleotidsequenzen aktiviert in Endothelzellen, glatten Muskelzellen, quergestreiften Muskelzellen, Makrophagen, Lymphozyten, Tumorzellen, Leberzellen, Leukämiezellen und Gliazellen oder von Promotorsequenzen der Viren HBV, HCV, HSV, HPV, EBV, HTLV oder HIV.

19. Nukleinsäurekonstrukt nach einem der vorhergehenden Ansprüche, dadurch gekennzeichnet, daß es sich bei dem Transgen (b) um ein Strukturgen handelt, das für ein Enzym kodiert, welches eine Vorstufe eines Pharmakons in ein Pharmakon spaltet.

20. Nukleinsäurekonstrukt nach einem der vorhergehenden Ansprüche, dadurch gekennzeichnet, daß es sich bei dem Transgen b) um ein Strukturgen handelt, das für ein Ligand-Enzym-Fusionsprotein kodiert.

21. Nukleinsäurekonstrukt nach Anspruch 20, dadurch charakterisiert, daß der Ligand an proliferierende Endothelzellen bindet und ausgewählt ist aus einer Gruppe umfassend Antikörper oder deren Fragmente, endständig Mannose-haltige Proteine, Cytokine wie IL-1 oder TNF, Wachstumsfaktoren wie PDGF, G-FGF, VEGF, TGFβ oder Adhäsionsmoleküle wie SLex, LFA-1, MAC-1, LECAM-1 oder VLA-1.

22. Nukleinsäurekonstrukt nach Anspurch 20, dadurch charakterisiert, daß der Ligand an Tumorzellen bindet.

23. Isolierte Zelle, dadurch gekennzeichnet, daß sie ein Nukleinsäurekonstrukt nach einem der Ansprüche 1 bis 22 enthält.

24. Verwendung eines Nukleinsäurekonstrukts nach einem der Ansprüche 1 bis 22 oder einer Zelle nach Anspruch 23 zur Bereitstellung eines Heilmittels zur Behandlung einer Erkrankung.

25. Verwendung eines Nukleinsäurekonstrukts nach einem der Ansprüche 1 bis 22 oder einer isolierten Zelle gemäß ♂Anspruch 23 zur Behandlung einer Erkrankung, wobei die Bereitstellung des Heilmittels die Einführung des Nukleinsäurekonstrukts in eine Zielzelle umfaßt.

26. Verwendung nach Anspruch 25, dadurch gekennzeichnet, daß die Erkrankung mit übermäßiger Zellvermehrung einhergeht.

27. Verwendung gemäß Anspruch 24 bis 26, dadurch gekennzeichnet, daß die Bereitstellung des Heilmittels die Überführung eines Nukleinsäurekonstruktes gemäß einem der Ansprüche 1 bis 22 in eine Form umfaßt, die die Einführung des Nukleinsäurekonstrukts in die Zielzelle ermöglicht.
